# EUROPEAN PATENT SPECIFICATION

(11) **EP 0 992 011 B1**
(45) Date of publication and mention of the grant of the patent: **28.08.2002**
(21) Application number: 98931493.5
(22) Date of filing: 23.06.1998
(51) Int. Cl.: G06F 17/50, G07F 17/30

(54) **METHOD AND APPARATUS FOR CONFORMATIONALLY ANALYZING MOLECULAR FRAGMENTS**
VERFAHREN UND GERÄT ZUR KONFORMATIONELLEN ANALYSE VON MOLEKULARFRAGMENTEN
PROCEDE ET APPAREIL D'ANALYSE CONFORMATIONNELLE DE FRAGMENTS MOLECULAIRES

(30) Priority: 24.06.1997 US 48944 P
(43) Date of publication of application: 12.04.2000
(73) Proprietor: Dupont Pharmaceuticals Research Laboratories, Inc., San Diego, CA 92121 (US)
(72) Inventor: SMELLIE, Andrew, S., Gilroy, CA 95020 (US); TEIG, Steven, L., Palo Alto, CA 94301 (US)
(74) Representative: Moon, Donald Keith
(86) International application number: US9813014
(87) International publication number: WO98059306

(56) References cited:
- WO-A-97/14106
- LEACH ET AL: "A combined model-building and distance geometry approach to automated conformational analysis and search" JOURNAL OF CHEMICAL INFORMATION AND COMPUTER SCIENCES., vol. 32, 1992, pages 379-385, XP002083947 WASHINGTON US
- LEACH ET AL: "Automated conformational analysis : algorithms for the efficient construction of low-energy conformations" JOURNAL OF COMPUTER-AIDED MOLECULAR DESIGN, vol. 4, 1990, pages 271-282, XP002083948 cited in the application
- SMELLIE ET AL : "Analysis of conformation coverage. 1. Validation and estimation of coverage" JOURNAL OF CHEMICAL INFORMATION AND COMPUTER SCIENCES., vol. 35, 1995, pages 285-294, XP002083949 WASHINGTON US
- SADOWSKI ET AL: "From atoms and bonds to three-dimensional atomic coordinates : automatic model builders" CHEMICAL REVIEWS., vol. 93, 1993, pages 2567-2581, XP002083950 EASTON US cited in the application

## Description

A portion of the disclosure of this patent document contains material which is subject to copyright protection. The copyright owner has no objection to the facsimile reproduction by anyone of the patent document or the patent disclosure as it appears in the Patent and Trademark Office patent file or records, but otherwise reserves all copyright rights whatsoever.

The present invention relates generally to the determination of molecular structure of chemical compounds by using computer based methods to analyze subspecies of the molecule, then combining the results from these analyses to determine the properties of the molecule.

Molecules have one or more three-dimensional structures. A molecule's structure determines its chemical, physical and bio-active properties. Scientists use a set of convenient parameters, such as bond length, bond angle and torsion angles, to describe the organization of atoms within a molecule that give rise to its molecular structure.

Researchers in the pharmaceutical field, for example, have sought for some time for a way to systematically analyze molecular structures of chemical compounds in order to determine their suitability as medicines. A conformation is the spatial arrangement of the atoms in a molecule at any point in time that results from rotation of parts of the molecule about covalent bonds and the "bending" of bond angles. Researchers in other fields also desire to search for chemical compounds having desirable attributes by analyzing fragments of molecules with a computer based method, rather than subjecting samples of the compounds to chemical analyses in a laboratory.

In A. Smellie et al., Journal of Chemical Information & computer Sciences 35, 1995, pages 285-294, there is described a method for determining the possible conformation of a molecule which includes decomposing the molecule into a number of fragments and determining for each fragment a plurality of possible conformers chosen so as to minimize torsional strain and steric interactions and combining the possible conformers in a test molecule in which the overall conformation is considered, conformations of the molecule determined as having that the lowest energy corresponding to possible conformations of the molecule.

What is needed is a method of determining a conformation for a molecule based upon information about the structure of its constituents.

The present invention provides techniques for improved automated determination of molecular information. More particularly, the present invention provides a method for-analyzing the structure of compounds.

According to an embodiment of the present invention there is provided, a computer based method for determining a conformation for a molecular structure comprising the steps of:
automatically decomposing the molecule into a plurality of partially overlapping and normalized fragments, each fragment having a limited maximum number of rotatable bonds, each bond having a number of possible torsion angles;
determining for each of said fragments one or more possible conformers thereof, torsion angles of each possible conformer having values that maximize a plurality of determined minimum energy difference levels;
determining for each of said fragments one or more candidate conformers, said candidate conformers having a range of likely torsion angles broader than the range of torsion angles of said possible conformers and obtained by correspondingly increasing by a predetermined amount said maximized minimum energy difference levels;
for each fragment of the molecule selecting and combining one or more candidate conformers to produce a low energy conformation for the molecular structure;
said method being characterized in that for each one of said partially overlapping fragments of the molecule a corresponding table is provided having a plurality of columns and rows and storing values defining said likely torsion angles of said one or more candidate conformers, each column representing a rotatable bond, each row representing one of said candidate conformers having a possible low-energy conformation of the fragment;
said step of selecting and combining is performed by:
   examining the tables corresponding to a pair of partially overlapping fragments and selecting rows that contain the same values for those rotatable bonds shared by the pair; creating an intersection table the rows of which combine the values of said selected rows:
      likewise examining said intersection table and the table corresponding to another partially overlapping fragment, thereby creating a further intersection table;
      repeating the above step until all tables for all fragments in the molecule have been examined;
      the rows of the finally obtained intersection table representing said low energy conformation for the molecular structure.

Some embodiments will also include a step of searching for one or more conformers fragments in a library. If the fragment is found in the library, then its corresponding conformers are read from the library. Otherwise, the method includes an additional step of storing the fragment and conformer information it produces in the determining step into the library for subsequent analyses.

In another aspect the present invention provides, a computer programming product as set out in appended claim 14.

In a yet further aspect of the present invention there is provided, an apparatus for determining a conformation for a molecular structure comprising:
means for automatically decomposing the molecule into a plurality of partially overlapping and normalized fragments, each fragment having a limited maximum number of rotatable bonds, each bond having a number of possible torsion angles;
means for determining for each of said fragments one or more possible conformers thereof, torsion angles of each possible conformer having values that maximize a plurality of determined minimum energy difference levels;
means for determining for each of said fragments one or more candidate conformers, said candidate conformers having a range of likely torsion angles broader than the range of torsion angles of said possible conformers and obtained by correspondingly increasing by a predetermined amount said maximized minimum energy difference levels;
means for selecting and combining for each fragment of the molecule one or more candidate conformers to produce a low energy conformation for the molecular structure;
said apparatus being characterized by:
   means for providing for each one of said partially overlapping fragments of the molecule a corresponding table having a plurality of columns and rows and storing values defining said likely torsion angles of said one or more candidate conformers, each column representing a rotatable bond, each row representing one of said candidate conformers having a possible low-energy conformation of the fragment;
   said means for selecting and combining including :
      means for examining the tables corresponding to a pair of partially overlapping fragments and selecting rows that contain the same values for those rotatable bonds shared by the pair, thereby creating an intersection table the rows of which combine the values of said selected rows;
      means for likewise examining said intersection table and the table corresponding to another partially overlapping fragment, thereby creating a further intersection table;
      means for repeating the above step until all tables for all fragments in the molecule have been examined;
      the rows of the finally obtained intersection table representing said low energy conformation for the molecular structure.

Numerous benefits are achieved by way of the present invention over conventional techniques. In some embodiments, the present invention provides more automated methods of analyzing molecular structures using a computer than many of the manual techniques heretofore known. The present invention can also provide a library of fragments which is without theoretical limit as to size or scope. Some embodiments according to the invention can consider entire molecular contexts in determining a molecular structure based upon fragment conformers. Select embodiments according to the invention may be more robust, than those using known techniques. These and other benefits are described throughout the present specification and more particularly below.

The invention will be better understood upon reference to the following detailed description and its accompanying drawings.
Fig. 1A depicts a simplified block diagram of a representative hardware embodiment according to the invention;
Fig. 1B depicts a functional perspective of the representative hardware embodiment according to the invention;
Figs. 2A-2E depict perspective views of a representative molecular structure having various conformations;
Fig. 3 depicts a graph of potential energy of a representative molecular system through the course of a rotation about a particular molecular bond;
Figs. 4A-4D depict flowcharts of representative processing steps in select embodiments according to the invention;
Fig. 5 depicts an example molecule;
Fig. 6 depicts an example fragment molecule of the molecule in Fig. 5;
Fig. 7 depicts a graph representation of the molecule of Fig. 5 with its molecular structure shown as a collection of nodes and edges;
Figs. 8A-8F depict possible fragments of the graph of Fig. 7;
Fig. 9 depicts a hypothetical molecular structure being broken into overlapping fragments;
Fig. 10 depicts conformation sets for each of fragments of Fig. 9;
Fig. 11 depicts the intersection of conformation sets of Fig. 10;
Fig. 12 depicts a sample molecule containing a ring structure;
Fig. 13 depicts the demarcation of a ring within the molecule of Fig. 12;
Fig. 14 depicts some of the fragments that are formed during decomposition of the molecule of Fig. 12;
Fig. 15 depicts tables used in conformational analysis and intersection of ring nodes;
Fig. 16 depicts an example molecular structure computationally decomposed into a plurality of fragments; and

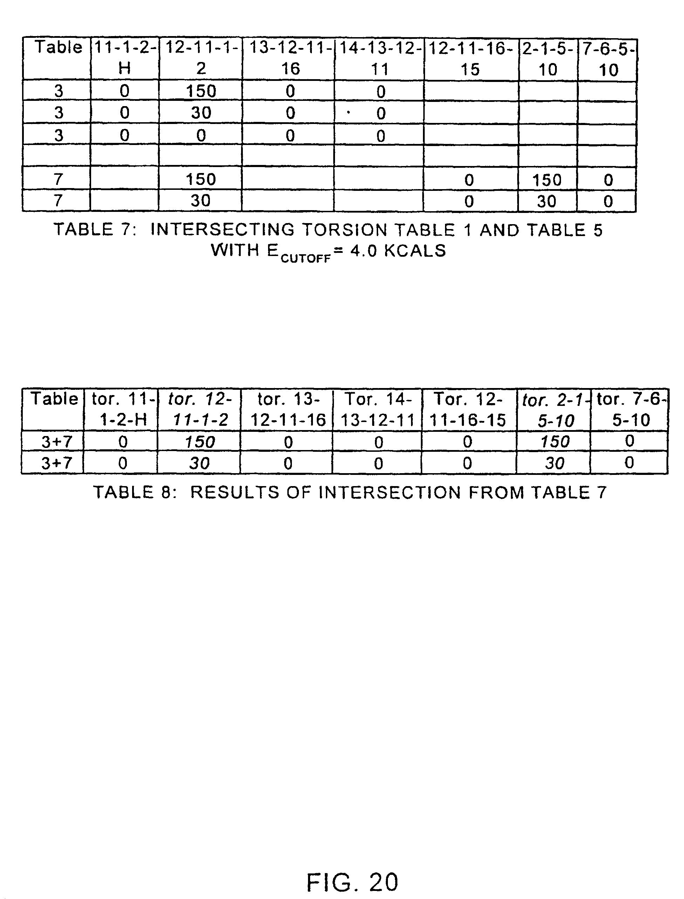

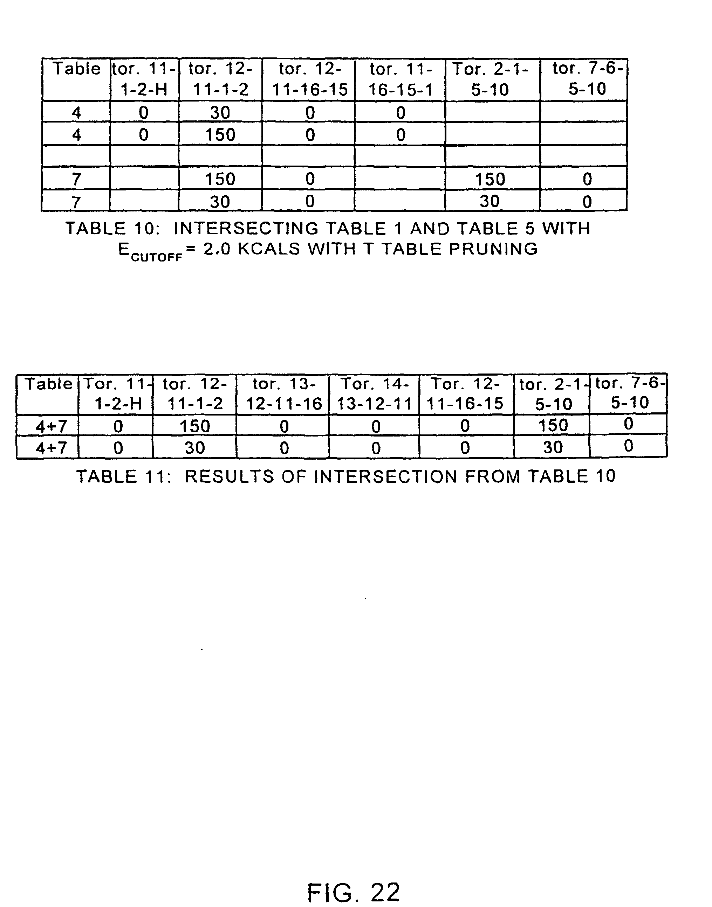

Figs. 17-22 depict simplified representative tables used in conformational analysis and intersection.

### 1.0 Introduction

The present invention provides techniques for determining conformers of molecules based upon information about component molecular fragments. Systems according to the present invention enable researchers and scientists to identify promising candidate compounds in the search for new and better substances.

### 1.1 Hardware Overview

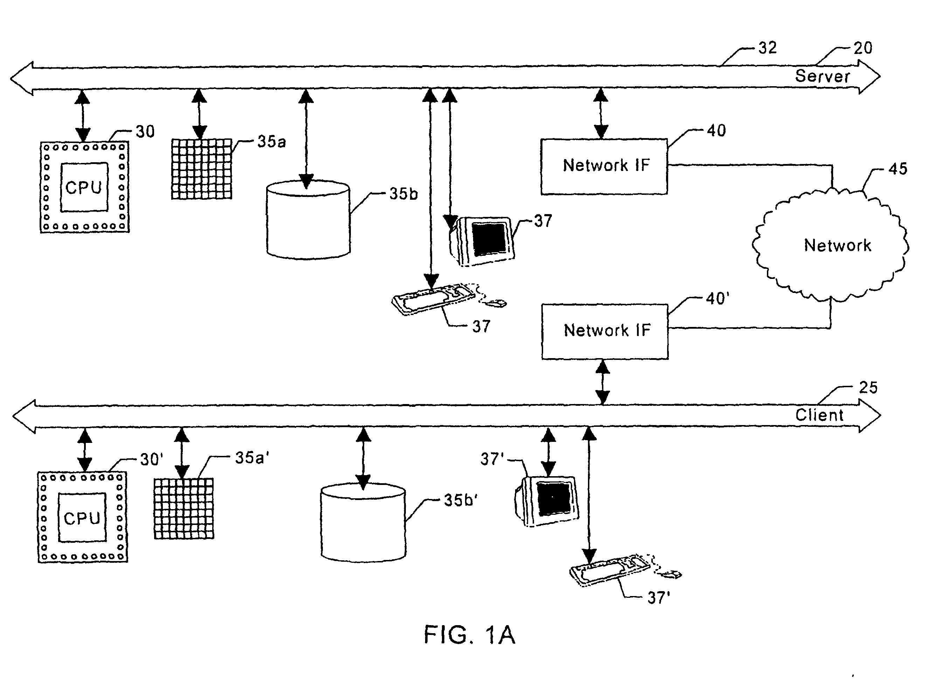

The method for determining a molecule's conformers is implemented in the C++ programming language and is operational on a computer system such as shown in Fig. 1A. This invention may be implemented in a client-server environment, but a client-server environment is not essential. Fig. 1A shows a conventional client-server computer system that includes a server 20 and numerous clients, one of which is shown as client 25. The use of the term "server" is used in the context of the invention, wherein the server receives queries from (typically remote) clients, does substantially all the processing necessary to formulate responses to the queries, and provides these responses to the clients. However, server 20 may itself act in the capacity of a client when it accesses remote databases located at another node acting as a database server.

The hardware configurations are in general standard and will be described only briefly. In accordance with known practice, server 20 includes one or more processors 30 that communicate with a number of peripheral devices via a bus subsystem 32. These peripheral devices typically include a storage subsystem 35, comprised of memory subsystem 35a and file storage subsystem 35b. Storage subsystem 35 is disposed to hold computer programs (e.g., code or instructions) and data. Other peripheral devices include a set of user interface input and output devices 37, and an interface to outside networks, which may employ Ethernet, Token Ring, ATM, IEEE 802.3, ITU X.25, Serial Link Internet Protocol (SLIP) or the public switched telephone network. This interface is shown schematically as a "Network Interface" block 40. It is coupled to corresponding interface devices in client computers via a network connection 45.

Client 25 has the same general configuration, although typically with less storage and processing capability. Thus, while the client computer could be a terminal or a low-end personal computer, the server computer is generally a high-end workstation or mainframe, such as a SUN SPARC™ server. Corresponding elements and subsystems in the client computer are shown with corresponding, but primed, reference numerals.

The user interface input devices typically includes a keyboard and may further include a pointing device and a scanner. The pointing device may be an indirect pointing device such as a mouse, trackball, touch pad, or graphics tablet, or a direct pointing device such as a touch screen incorporated into the display. Other types of user interface input devices, such as voice recognition systems, are also possible.

The user interface output devices typically include a printer and a display subsystem, which includes a display controller and a display device coupled to the controller. The display device may be a cathode ray tube (CRT), a flat-panel device such as a liquid crystal display (LCD), or a projection device. The display controller provides control signals to the display device and normally includes a display memory for storing the pixels that appear on the display device. The display subsystem may also provide non-visual display such as audio output.

The memory subsystem typically includes a number of memories including a main random access memory (RAM) for storage of instructions and data during program execution and a read only memory (ROM) in which fixed instructions are stored. In the case of Macintosh-compatible personal computers the ROM would include portions of the operating system; in the case of IBM-compatible personal computers, this would include the BIOS (basic input/output system).

The file storage subsystem provides persistent (non-volatile) storage for program and data files, and typically includes at least one hard disk drive and at least one floppy disk drive (with associated removable media). There may also be other devices such as a CD-ROM drive and optical drives (all with their associate removable media). Additionally, the computer system may include drives of the type with removable media cartridges. The removable media cartridges may, for example be hard disk cartridges, such as those marketed by SyQuest and others, and flexible disk cartridges, such as those marketed by Iomega. One or more of the drives may be located at a remote location, such as in a server on a local area network or at a site of the Internet's World Wide Web.

In this context, the term "bus subsystem" is used generically so as to include any mechanism for letting the various components and subsystems communicate with each other as intended. With the exception of the input devices and the display, the other components need not be at the same physical location. Thus, for example, portions of the file storage system could be connected via various local-area or wide-area network media, including telephone lines. Similarly, the input devices and display need not be at the same location as the processor, although it is anticipated that the present invention will most often be implemented in the context of PCs and workstations.

Bus subsystem 32 is shown schematically as a single bus, but a typical system has a number of buses such as a local bus and one or more expansion buses (e.g., ADB, SCSI, ISA, EISA, MCA, NuBus, or PCI), as well as serial and parallel ports. Network connections are usually established through a device such as a network adapter on one of these expansion buses or a modem on a serial port. The client computer may be a desktop system or a portable system.

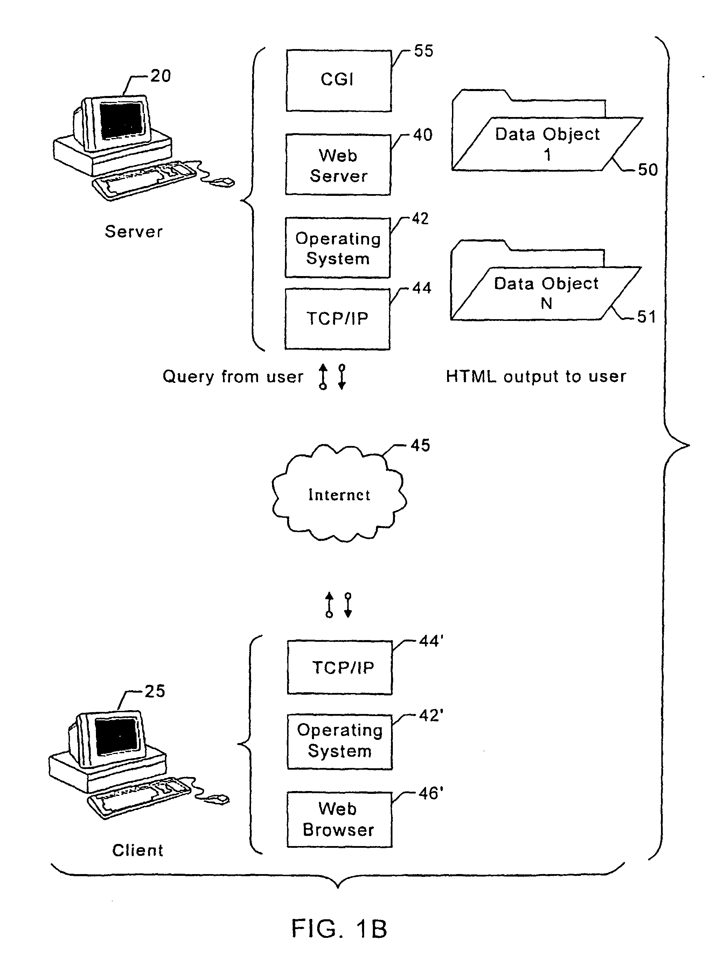

Fig. 1B is a functional diagram of the computer system of Fig. 1A. Fig. 1B depicts a server 20, and a representative client 25 of a multiplicity of clients that may interact with the server 20 via the internet 45 or any other communications method. Blocks to the right of the server are indicative of the processing components and functions that occur in the server's program and data storage indicated by block 35a in Fig. 1A. A TCP/IP "stack" 44 works in conjunction with Operating System 42 to communicate with processes over a network or serial connection attaching Server 20 to internet 45. Web server software 46 executes concurrently and cooperatively with other processes in server 20 to make data objects 50 and 51 available to requesting clients. A Common Gateway Interface (CGI) script 55 enables information from user clients to be acted upon by web server 46, or other processes within server 20. Responses to client queries may be returned to the clients in the form of a Hypertext Markup Language (HTML) document outputs which are then communicated via internet 45 back to the user.

Client 25 in Fig. 1B possesses software implementing functional processes operatively disposed in its program and data storage as indicated by block 35a' in Fig. 1A. TCP/IP stack 44', works in conjunction with Operating System 42' to communicate with processes over a network or serial connection attaching Client 25 to internet 45. Software implementing the function of a web browser 46' executes concurrently and cooperatively with other processes in client 25 to make requests of server 20 for data objects 50 and 51. The user of the client may interact via the web browser 46' to make such queries of the server 20 via internet 45 and to view responses from the server 20 via internet 45 on the web browser 46'.

### 2.0 Torsion and Conformation

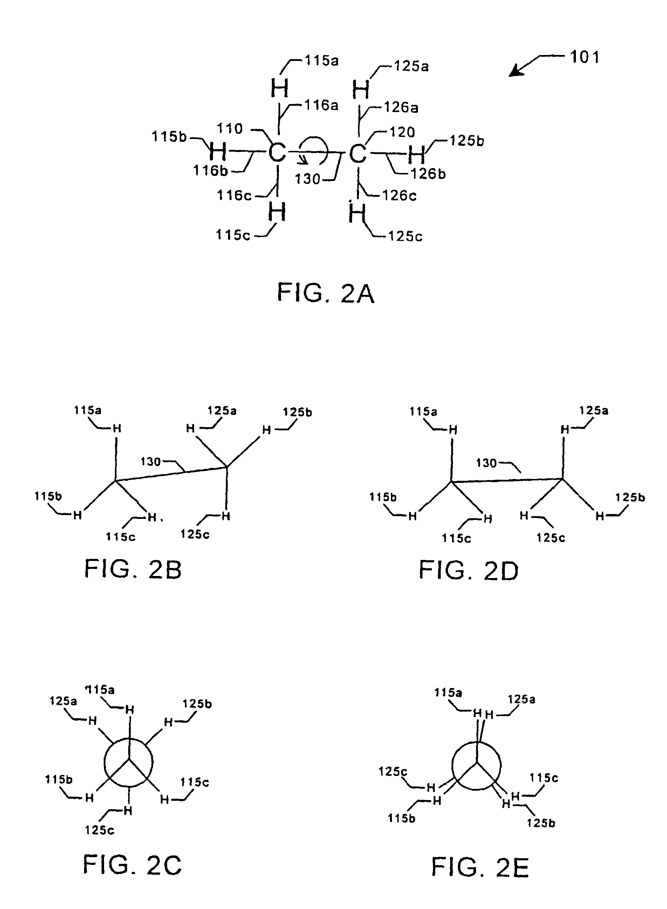

Fig. 2A depicts a model of a molecule 101, in this example an ethane molecule. Ethane molecule 101 comprises the carbon atoms 110 and 120 and hydrogen atoms 115a, 115b, 115c, 125a, 125b, and 125c. Carbon atom 110 is bound to hydrogen atoms 115a, 115b, and 115c via single bonds 116a, 116b, and 116c, respectively, forming a tetrahedron with carbon atom 110 at its center. Carbon atom 120 is bound to hydrogen atoms 125a, 125b, and 125c via single bonds 126a, 126b, and 126c, respectively, forming a second tetrahedron. Carbon atom 110 is bound to carbon atom 120 via single bond 130. Single bond 130 is subject to torsion, whereby each tetrahedron rotates relative to the other

Figs. 2B through 2E depict two conformations of ethane molecule 101. Fig. 2B depicts a perspective view of molecule 101 in a staggered conformation. In the staggered conformation, bond 130 is subjected to a torsion such that each hydrogen atom 115a, 115b, and 115c is positioned the maximum angular distance from each hydrogen atom 125a, 125b and 125c. Fig. 2C depicts a Newman projection of molecule 101 in a staggered conformation. It will be noted that, in the staggered conformation, each hydrogen atom 115a, 115b, and 115c is positioned 60 degrees from each hydrogen atom 125a, 125b and 125c in the plane perpendicular to the longitudinal axis of bond 130. This angle is referred to as the "dihedral angle" or the "angle of torsion."

Fig. 2D depicts a perspective view of molecule 101 in an eclipsed conformation. In the eclipsed conformation, bond 130 is subjected to a torsion such that each hydrogen atom 115a, 115b, and 115c is positioned the minimum angular distance from each hydrogen atom 125a, 125b and 125c. Fig. 2E depicts a Newman projection of molecule 101 in an eclipsed conformation. It will be noted that, in the eclipsed conformation, each hydrogen atom 115a, 115b, and 115c is positioned coincident to the same position of each hydrogen atom 125a, 125b and 125c in the plane perpendicular to the longitudinal axis of bond 130. That is, the angle of torsion is zero degrees.

Torsions are an example of an internal coordinate representation of the molecule whereby with knowledge of the torsion angle about the bond 130 and coordinates of each tetrahedron centered at carbon atoms 110 and 120, coordinates for all the atoms may be determined. In general, the representation of a conformation by its internal coordinates can be used to generate atomic coordinates for each atom in the molecule (modulo rotation and translation). There are many types of internal coordinates, well-known to those skilled in the art, that can be used in conjunction with the invention, but, in the preferred embodiment, torsion angles are the internal coordinate system used.

In the course of a complete 360-degree rotation, the molecule alternates between three instances of the staggered conformation and three instances of eclipsed conformation. The three instances of staggered conformation occur at 60 degrees, 180 degrees and 300 degrees. The three instances of eclipsed conformation occur at zero (or 360) degrees, 120 degrees, and 240 degrees.

Experimental observations have shown that torsion angles are easier to change than bond angles, which in turn are easier to deform than bond lengths. This observation lead to the "fixed valence approximation" in which bond lengths and bond angles are assumed to be invariant, leaving only torsional angles as determiners of a molecule's structure. Even plastic molecular models hold bond angle and bond length fixed.

Generally, the number of conformers to be determined for a particular molecule may be calculated by the expression *N* = *s*^{*b*}, where *s* is the number of samples of angular torsion to be examined for a bond, and b is the number of bonds whose torsion is to be sampled. For example, to calculate conformers for ethane molecule 101, given a sampling at 0, 60, 120, 180, 240 and 300 degrees, six samples are made of a single bond. Therefore, the number of conformers is *N* = *s*^{*b*} = 6¹ = 6. Calculating conformers for a simple molecule such as ethane is computationally inexpensive. However, as the complexity of a molecule increases, the cost of calculating its conformers increases exponentially. For example, to calculate the conformers of a molecule having only 20 rotatable bonds and sampling each bond's torsion angle at 60-degree increments (i.e., six samples over 360 degrees of angle), N = *s*^{*b*} = 6²⁰ = 3,656,158,440,062,980 conformers must be calculated. Increasing the number of samples to every 10 degrees (i.e., 36 samples over 360 degrees of angle) on a molecule having 100 rotatable bonds would require the generation of 4.268 x 10¹⁵⁵ conformers. The calculation and evaluation of potential preferred conformers as required in present-day drug discovery using conventional systematic searching methods clearly is beyond the capabilities of even the fastest available computers. An alternative that has been tried is the use of random selection to find promising conformers by weighted chance, and then exploring similar conformers systematically. This approach, however, by its very nature risks missing useful conformers that were not randomly selected.

Additional information regarding conformation and torsion may be found in Eliel, Stereochemistry of Carbon Compounds, Ch. 6, pp. 124-179 (1962), the disclosure of which is hereby incorporated by reference.

### 3.0 Potential Energy of a Conformer

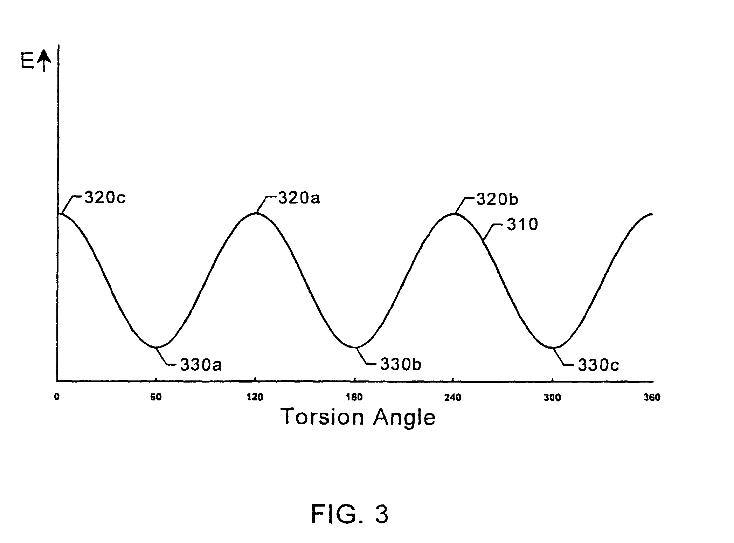

Fig. 3 depicts the potential energy of the ethane molecule of Fig. 2A through the course of a 360-degree rotation. Plotted line 310 graphically depicts the potential energy as a function of the angle of torsion through the course the rotation. Each point on the plotted line corresponds to a possible conformation of the ethane molecule 101. The points 330a, 330b and 330c correspond to the torsion angles of 60, 180, and 300 degrees, respectively, and represent the three staggered conformations. The points 320a, 320b, and 320c correspond to the torsion angles of 120, 240 and 360 degrees, respectively, and correspond to the three eclipsed conformations. It will be seen that the three staggered conformations represent the conformations at the energy minima and are therefore the most preferred conformations.

### 4.0 Process Flow of the Specific Embodiments

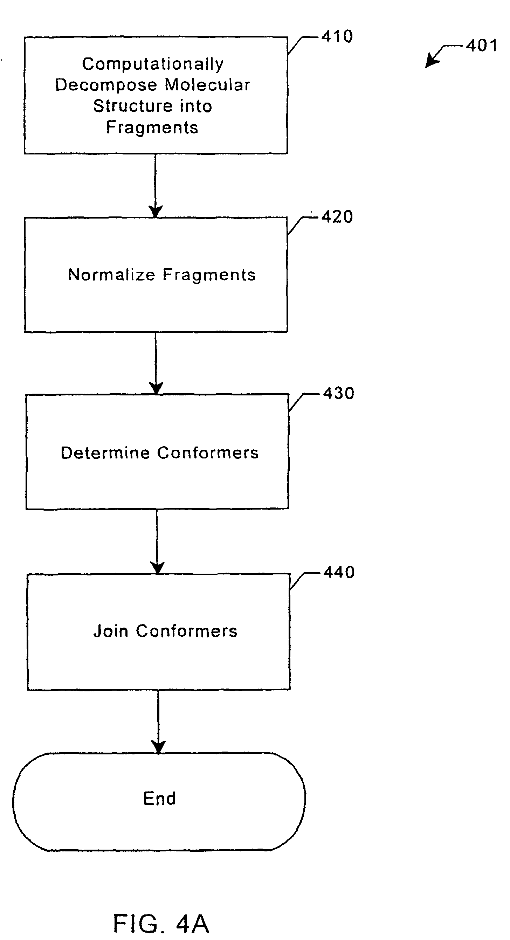

Fig. 4A depicts a flowchart 401 of simplified process steps in a particular representative embodiment according to the invention for determining a conformation for a molecular structure. In a step 410, the molecular structure is computationally decomposed into a plurality of fragment molecular structures. Then, in a step 420, each one of the plurality of fragment molecular structures is normalized in order to form a plurality of normalized fragments. Subsequently, in a step 430, at least one of a plurality of conformers is determined for each normalized fragment produced in step 420. Finally, in a step 440, at least a first conformer and a second conformer determined in step 420, are combined in order to derive a conformation for the entire molecular structure.

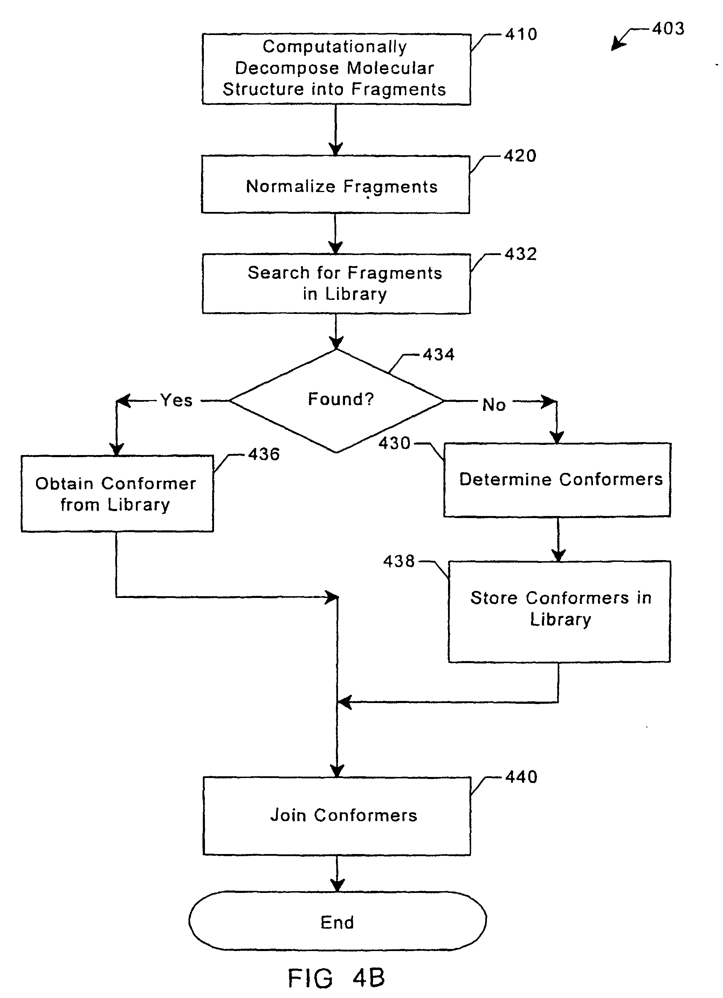

Fig. 4B depicts a flowchart 403 of simplified process steps in another embodiment according to the invention. In this embodiment, in a step 410, the molecular structure is computationally decomposed into a plurality of fragment molecular structures. Then, in a step 420, each one of the plurality of fragment molecular structures is normalized in order to form a plurality of normalized fragments. Next, in a step 432, a search is performed for one or more normalized fragments in a library. Then, in a decisional step 434, a determination is made whether the fragment was located in the library. If the fragment is found in the library, then in a step 436, its corresponding conformers are read from the library. Otherwise, in a step 430, at least one of a plurality of conformers is determined for each normalized fragment produced in step 420. Next, in a step 438, the fragment and conformer information produced in step 430 is stored in the library for subsequent analyses. Finally, in a step 440, at least a first conformer and a second conformer determined in step 430, or found in the library in step 436 are combined in order to derive a conformation for the entire molecular structure.

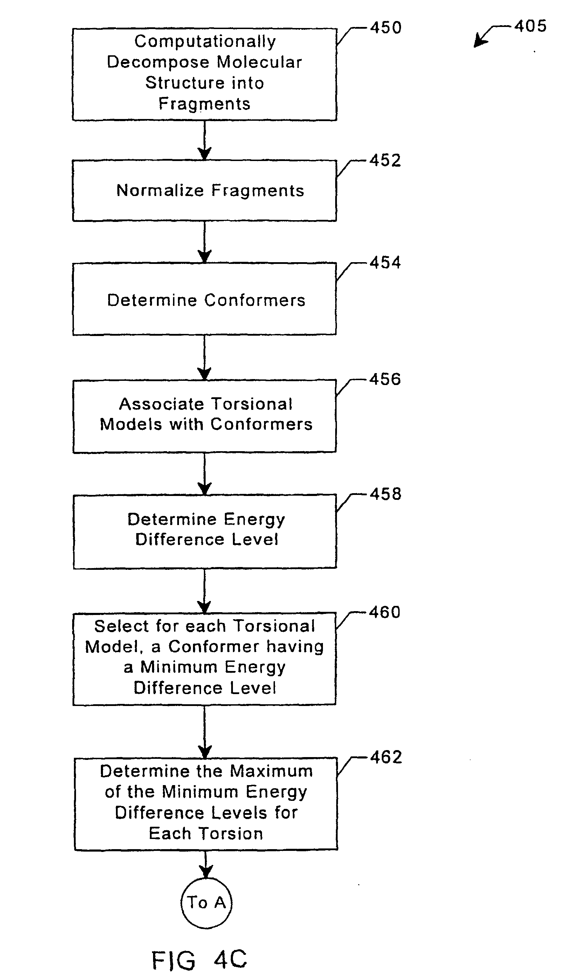

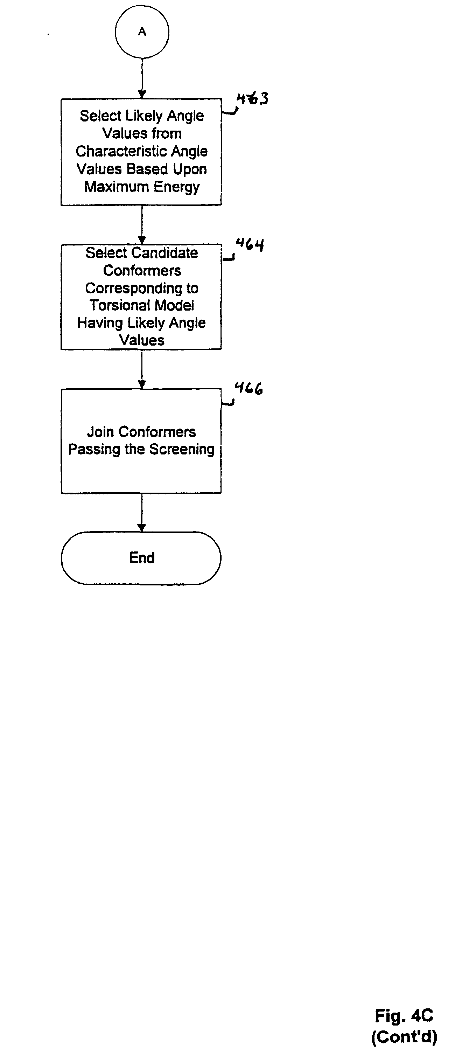

Fig. 4C depicts a flowchart 405 of simplified process steps in another embodiment according to the invention. In this embodiment, in a step 450, the molecular structure is computationally decomposed into a plurality of fragment molecular structures. Then, in a step 452, each of the plurality of fragments is normalized to form a plurality of normalized fragments. Next, in a step 454, at least one of a plurality of conformers is determined for each normalized fragment determined in step 452. Each conformer in the plurality of conformers has at least one of a plurality of chemical bond representations, and each chemical bond representation interconnects at least two atoms in the eonformer. In a step 456, a plurality of internal coordinates (i.e., torsional models) is associated with each conformer, each of the torsional models possessing a plurality of characteristic angle values corresponding to each chemical bond representation in the conformer. Then, in a step 458, for each conformer, an energy difference level is determined. This energy difference level represents an incremental amount of potential energy above a specified nominal energy value required to maintain each chemical bond representation at each of the plurality of characteristic angle values in the torsional model. Next, in a step 460, for each torsional model, for each characteristic angle value, a conformer having a corresponding minimum energy difference level, is selected. The corresponding minimum energy difference level is selected from the energy difference levels computed for each of the conformers in each of the fragments. In a step, 462, a maximum energy difference value is determined for each characteristic angle value from among the corresponding minimum energy difference levels. This forms a plurality of maximum energy difference values corresponding to the plurality of conformers. Then, in a step 463, a plurality of likely angle values is selected from among the plurality of characteristic angle values such that the plurality of likely angle values in the torsional models corresponds to a nominal energy value. This nominal energy value is determined by adding an incremental energy to the maximum energy difference values formed in step 462. Next, in a step 464, a plurality of candidate conformers is selected from the plurality of conformers, each candidate conformer corresponds to a torsional model having at least one characteristic angle value in the plurality of likely angle values determined in preceding step 463. Finally, in a step 466, at least two candidate conformers chosen from the plurality of candidate conformers are combined to produce a low energy conformation for the molecular structure. Candidate conformers are chosen such that the energy difference level of each of the candidate conformers is less than a specified cutoff energy level. Further, the candidate conformers are chosen such that they have at least one common torsional model.

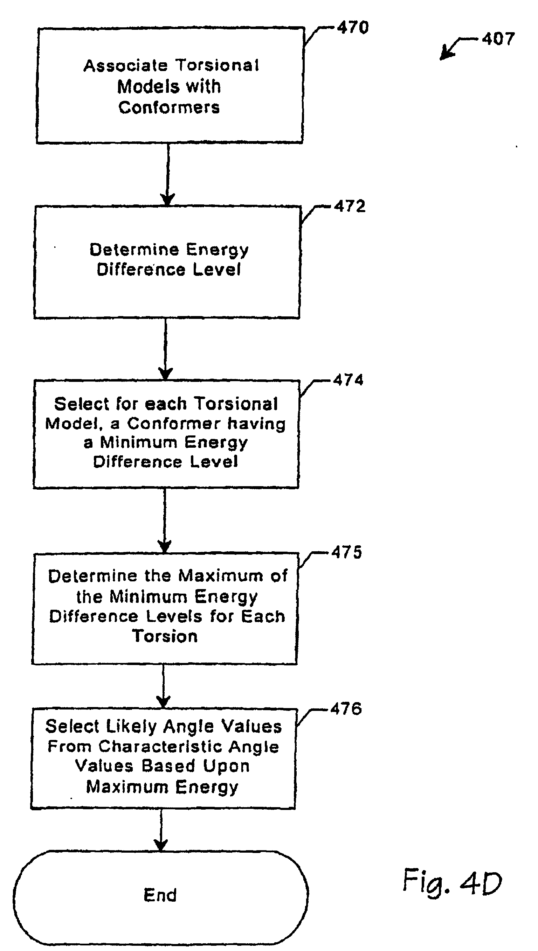

Fig. 4D depicts a flowchart 407 of simplified process steps in another embodiment according to the invention. In this embodiment, in a step 470, a plurality of internal coordinates (i.e. torsional models) is associated with each conformer in a plurality of conformers. Each of the torsional models possesses a plurality of characteristic angle values corresponding to each chemical bond representation in the conformer. Then, in a step 472, for each conformer, an energy difference level is determined. This energy difference level represents an incremental amount of potential energy above a specified nominal energy value required to maintain each chemical bond representation at each of the plurality of characteristic angle values in the torsional model. Next, in a step 474, for each torsional model, for each characteristic angle value, a conformer having a corresponding minimum energy difference level, is selected. The corresponding minimum energy difference level is selected from the energy difference levels computed for each of the conformers in each of the fragments. In a step 475, a maximum energy difference value is determined for each characteristic angle value from among the corresponding minimum energy difference levels. This forms a plurality of maximum energy difference values corresponding to the plurality of conformers. Then, in a step 476, a plurality of likely angle values is selected from among the plurality of characteristic angle values such that the plurality of likely angle values in the torsional models corresponds to a nominal energy value. This nominal energy value is determined by adding an incremental energy to the maximum energy difference values formed in step 475.

### 4.1 Decomposition

Select embodiments according to the invention employ a process of computationally decomposing the molecule under analysis into a plurality of fragments. The fragment size is selected to allow for a reduced number of calculated conformers for the fragment, while still maintaining a sufficient length so that the fragment conformers produced accurately represent the possible configurations of that fragment within the entire molecule.

In the general case, it is not obvious how the molecular decomposition should be done. In other works, molecules have been fragmented at bonds which are obvious to those skilled in art. One such decomposition is that of polypeptides into amino acid sequences (described in Vasqez and Scheraga, Biopolymers, Vol 24, pp.1437-1447, which is incorporated herein by reference in its entirety for all purposes). Other decompositions have partitioned the molecule into rings and chains (see, for example, Sadowski and Gasteiger, Chem Rev., 1993, 93, pp 2567-2581 and Leach, Prout and Dolata, J.Comp.-Aided Mol.Des.,4(1990), pp271-282). Where such decompositions are not obvious, as is the case for general molecules, other fragmentation scheme must be considered.

A particularly useful fragment size is a fragment comprising up to 4 rotatable bonds. A fragment of this size includes the five atoms bound to the rotatable bonds, plus every atom present in the molecule that is bonded to any of the five atoms. Such a fragment is said to have a path length of length 7. At times in this specification, the process of moving, counting, measuring, etc., along a path from one atom or bond to the next is referred to as "walking" the fragment or path length.

In a fragment having a path-length of 7, four of the bonds are subject to rotation in a conformational analysis of the fragment. The remaining two bonds, while subject to rotation within the molecule, have little effect on the conformation of the fragment, and need not be rotated in a conformational analysis of the fragment. It is known that, for drug-sized molecules, the overwhelming number of close atomic contacts occur between atoms that are connected by a path length of 7 or closer. Thus, if conformers are constructed that eliminate steric clashes within a length 7, it is unlikely that a molecule made up of such conformers will have any steric clashes at all.

By limiting a fragment to consideration of up to four rotatable bonds, the number of potential conformers that need to be analyzed for the fragment is greatly reduced. As will be discussed below with respect to conformer generation, to generate a conformer for the fragment each bond is sampled either 8, 18, or 36 times, depending on the types of atom groups attached to the bond. The atom groups result from hybridization of atomic orbitals and is discussed further below. Therefore, exhaustive calculation of a particular fragment will generate only between 8' (4096) and 36' (1,679,616) potential conformer configurations for the fragment. This range of potential configurations is well within a practical computational capabilities of widely available and inexpensive computer systems.

The use of a fragment in which rotation around four bonds is analyzed therefore allows for a reduced number of calculated conformers for the fragment and yet has a sufficient length so that the fragment conformers produced accurately represent the possible configurations of that fragment within the entire molecule.

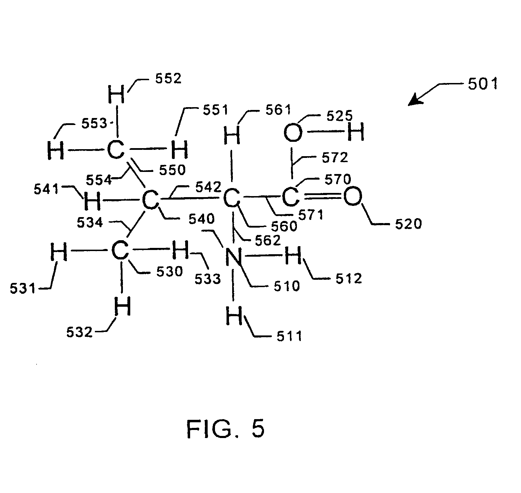

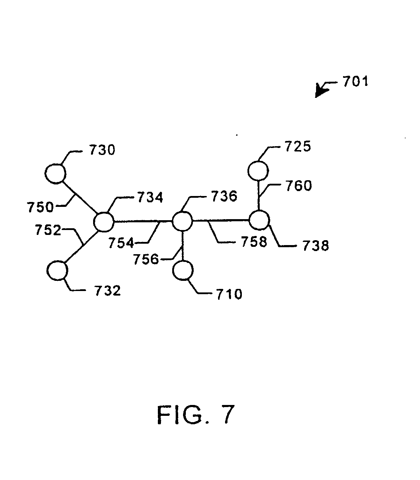

Fig. 7 depicts a graphic representation 701 of the molecule of Fig. 5 with its molecular structure described as a plurality of nodes and edges. A node represents a collection of atoms. An edge represents a freely rotatable bond between two atoms. Within a single node; no two atoms are connected by an edge. In other words, there are no freely rotatable bonds between any two atoms represented by a node. Nodes 710, 725, 730, 732, 734, 736, and 738 correspond to atoms 510, 525, 550, 530, 540, 560, and 570, and their related outlying univalent atoms, respectively. Edges 750, 752, 754, 756, 758 and 760 correspond to bonds 554, 534, 542, 562, 571 and 572, respectively. Because outlying bonds to univalent atoms are not significantly rotatable and will not be analyzed in a conformational analysis, the atoms bonded by these bonds are represented by the node that also represents the atom to which they are bound. For example, node 710 represents nitrogen atom 510 and hydrogen atoms 511 and 512, because bond 562 is rotatable and delimits the node.

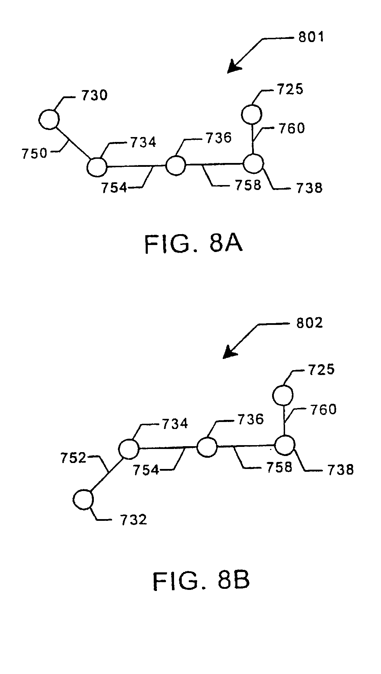

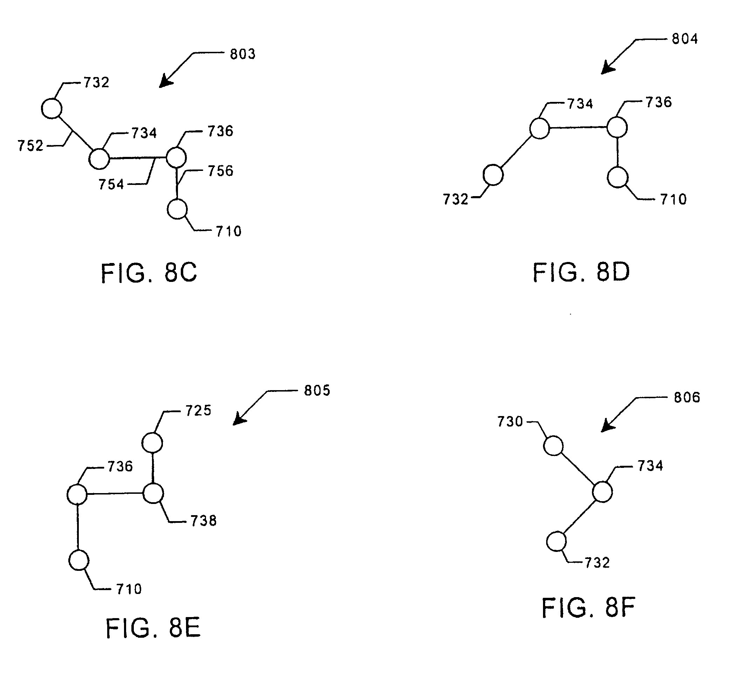

A molecule represented as a graph may be broken up into a series of paths, each path having a length equal to or less than a selectable maximum number of atoms, which may be five atoms or fewer in select embodiments. These atoms, combined with all atoms bonded to them, form the path that has a maximum path length of seven or fewer atoms. Figs. 8A through 8F depict six possible fragments of graph 701 having a length 7 or less. Fig. 8A depicts a first possible fragment 801, comprising nodes 730, 734, 736, 738, and 725 connected by edges 750, 754, 758, and 760. Fig. 8B depicts a second possible fragment 802, comprising nodes 732, 734, 736, 738 and 725 connected by edges, 752, 754, 758, and 760. Fig. 8C depicts a third possible fragment 803, comprising nodes 732, 734, 736 and 710 connected by edges 752, 754 and 756. Likewise, Figs. 8D through 8F depict additional possible fragments.

To further optimize the conformation generation process, fragments that are known not to provide additional information are excluded from further consideration. For example, in a scenario where a path of length 7 walks into a ring, walking more than two bonds around the ring means that, within the local context of the fragment, the ring constrains the end points of the path so they can never interact. The bonds that are in the ring are not freely rotatable, but are constrained by the conformations of the ring structure as a whole. The conformations of the path in isolation are constrained when a path intersects a ring. This interaction between paths and rings is explained more fully below.

### 4.2 Normalization

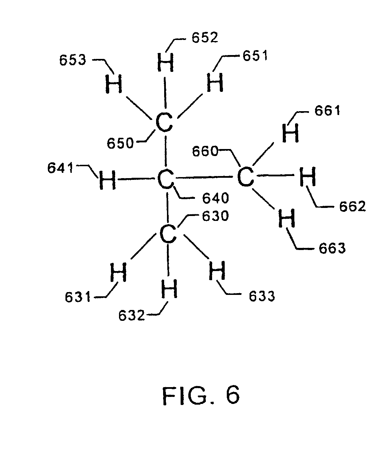

Once the fragments have been automatically generated from the decomposition of the molecule as described herein, the fragments are normalized to capture some of their context in the environment of the molecule. In general, a fragment in isolation will have a different conformational model than a fragment in context of a molecule because of the influence of the surrounding environment. In a present preferable embodiment, each fragment isolated from the molecule is stubbed, or *normalized*, to simulate the rest of the molecule environment. Consider the fragment 806 of Fig. 8F. This contains nodes 730, 734 and 732 which correspond to atoms 550, 540 and 530 (with their associated univalent atoms) respectively. This fragment is shown in Fig 6, where atoms 650, 640 and 630 correspond to original atoms 550, 540 and 530 respectively. Retaining atoms one level out means that atom 660 in Fig 6 corresponds to original atom 560. To simulate the environment of the whole molecule, and satisfy valency, hydrogen atoms 661, 662 and 663 are added to model atoms 561, 570 and 510 respectively. This embodiment normalizes the fragment by stubbing out the molecular environment with hydrogen. Other embodiments may not stub at all, or stub with an arbitrarily large fragment intended to simulate the rest of the molecule, without departing from the scope of the invention.

### 4.3 Conformer Generation

Select embodiments according to the invention include a step of determining a set of potential conformers for each fragment. A database can be employed to obtain sets of conformers that have been generated previously and to store sets of conformers that are being generated for the first time. Embodiments using the database include an additional step reducing each fragment to a text string that describes the atomic structure of the fragment for use as a database search key. The text string is put in canonical form so that the same text string is generated, even if there are variations in the fragment, such as reversed order of atoms, and so forth, that do not affect its conformational analysis. In this case, the generation of the canonical text string comprises of two steps: (1) placing the atoms of the fragment in a canonical order; (2) using this canonical order to generate a unique text string.

Canonical orderings are generated by first generating a simple hash key for each atom in the fragment. This key is formed by encoding the atom's simple properties (e.g., atomic number, charge, hybridization, aromaticity) to make the hash key. Each key is examined for unique atoms within the fragment. If any are found, they are placed in the canonical ordering in lexicographic order. If two or more atoms have the same simple hash key, their neighbors are recursively examined to update the hash key.
Then the keys are re-examined to look for unique atoms, which are placed in the canonical ordering. This process is repeated until all distinguishable atoms have been identified. If two atoms are truly the same in the fragment, they are arbitrarily given unique names.

Once a canonical ordering is generated, the canonical text string is generated by encoding each atom's simple properties and storing this encoding in canonical order. Then, for each atom in canonical order, all bonds from this atom ( in canonical order) are encoded and written to the canonical string. Thus, the canonical text string is formed by encoding information about the atoms and bonds in canonical order.

After canonical text string is formed, it is used as a key in a call to a database management system (DBMS) to retrieve a previously determined set of conformers for the fragment, as indicated by Step 436 in Fig. 4B. In the first iteration, there will generally be no previously stored conformers. As a result, the DBMS will indicate that the set was not found. Select embodiments may include a supply of conformational models of frequently used fragments.

For example, although fragment 801 and fragment 802 are different fragments within the molecule, they differ only in that, in fragment 801, node 730 represents atom 630, while in fragment 802, node 732 represents atom 632. However, both atom 630 and atom 632 are carbon atoms tetrahedrally bound to three hydrogen atoms. Therefore, a fragment conformation set calculated for fragment 801 is equally applicable to fragment 802. When it is necessary to determine a conformation set for fragment 802, the previously calculated conformation set form 801 may be used.

If no previously stored conformer set exists for the fragment, a conformer set is generated for the fragment. The conformer set may be generated using any prior art means. In one embodiment, the systematic search method is the preferable means for generating conformers. In systematic search, a fixed set of search positions is established for each rotatable bond. Then each combination of torsions is systematically searched. Coordinates of the atoms are generated for each combination of torsions, and the energy of the conformer is measured. Conformers having an energy value within N kcals of a global minimum energy value are retained. Energy level N is obtained by multiplying the number of rotatable bonds by a factor of 1.0-4.0. Systematic search methods are more fully described in Lipton and Still, *The Multiple Minimum Problem in Molecular Modelling. Tree Searching in Internal Coordinate Space,* Journal of Computational Chemistry, 1989, Vol.9, No.9,pp.343-55, which is incorporated herein by reference in its entirety for all purposes.

The systematic search is performed on a number of different degrees of torsion. By way of example, in a representative embodiment, the number of tests can be dependent upon the nature of the atom groups on either end of the bond subjected to torsion which in turn depends on hybridization of atomic orbitals that occur in bonding. For example, in a representative embodiment, if both atom groups are of order sp³, then about 18 samples are taken. If both atom groups are of order sp² with a single bond, then some 24 samples may be taken. If both atom groups are of order sp² with a double bond, then approximately 6 samples are taken. If one atom group is of order sp³ and the other is of order sp², then about 36 samples are taken. The number and position of the points sampled may be controlled by parameters that may be specified at execution time.

In the example representative embodiment, the sp³-sp³ combination, may be sampled at some major points, such as maybe three points, of approximately 60, 180, and 300 degrees, because these are low-energy areas for sp³-sp³ configuration. Samples may be taken at major points about 0, 120, and 240 degrees. In certain crowded molecules, an eclipsed configuration in one sp³-sp³ pair may result in a lower overall energy level for the molecule taken as a whole. In addition, samples can also be taken at minor points located anywhere from about -10 degrees to about +10 degrees about each major point. This additional sampling captures conformers that would not be of lowest energy if the bond were considered alone, but may be of lower energy due to the interactions of other atoms in the fragment. Consequently, for the sp³-sp³ combination, samples may be taken in groups of points. For example, three groups of three points each, may be taken at approximately 50, 60, and 70 degrees; 170, 180, and 190 degrees; 290, 300, and 310 degrees; 350, 0, and 10 degrees; 110, 120, and 130 degrees and 230, 240, and 250 degrees, for a total of 18 samples. Other sampling regimes employing differing number or points of samples may be used without departing from the scope of the invention.

Further, in the example representative embodiment, the sp²-sp³ combination may be sampled at some major points, such as maybe six points at approximately 0, 60, 120, 180, 240 and 300 degrees. Points may be selected because the point corresponds to a low-energy area for an sp²-sp³ configuration. Samples can also be taken at major points of about 30, 90, 150, 210, 270, and 330 degrees. In certain crowded molecules, these values may result in a lower overall energy in the context of the molecule. In addition, samples can be taken at minor points located anywhere from about -10 degrees to about + 10 degrees about each major point. This additional sampling captures conformers that would not be of lowest energy if the bond were considered alone, but may be of lower energy due to the interactions of other atoms in the fragment. For example, in the sp²-sp³ combination, samples may be taken in 12 groups of three points each. This sampling regime provides for sampling at approximately 350, 0, and 10 degrees; 20, 30, and 40 degrees; 50, 60, and 70 degrees; 80, 90, and 100 degrees; 110, 120, and 130 degrees; 140, 150, and 160 degrees; 170, 180, and 190 degrees; 200, 210, and 220 degrees; 230, 240, and 250 degrees; 260, 270, and 280 degrees; 290, 300, and 310 degrees and 320, 330, and 350 degrees, for a total of 36 samples. Other sampling regimes employing differing number or points of samples may be used without departing from the scope of the invention.

Further, in the example representative embodiment, the sp²-sp² combination joined by a single bond, may be sampled at some major points, such as maybe eight major points at approximately 0, 60, 120, 180, 240, and 300 degrees. These points are selected because they are low-energy areas for an sp²-sp² single bond configuration. Samples can also be taken at major points around 90 and 270 degrees. In certain crowded molecules, these values may result in a lower overall energy in the context of a molecule. In addition, samples can be taken at oh say 16 minor points located anywhere from about -10 degrees to about +10 degrees about each major point. For example, samples may be taken in 8 groups of three points each. Accordingly, samples could be taken at approximately 350, 0 and 10 degrees; 170, 180, and 190 degrees; 50, 60, and 70 degrees; 110, 120, and 130 degrees; 230, 240, and 250 degrees; 290, 300, and 310 degrees, 80, 90, and 100 degrees; and 260, 270, and 280 degrees, for a total of 24 samples. Other sampling regimes employing differing number or points of samples may be used without departing from the scope of the invention.

Further, in the example representative embodiment, the sp²-sp² combination joined by a double bond, may be sampled at some major points, such as 2 major points at approximately 0 and 180 degrees. These points are selected because they are low-energy areas for sp²-sp² double bond configuration. In addition, samples can be taken at 4 minor points located anywhere from about -10 degrees to about +10 degrees about each major point. For example, samples may be taken in 2 groups of 3 points each. Accordingly, samples could be taken at approximately 350, 0, and 10 degrees; and at 170, 180, and 190 degrees, for a total of 6 samples. Other sampling regimes employing differing number or points of samples may be used without departing from the scope of the invention.

Possible fragment conformers having bonds rotated in the amount indicated are calculated. If the calculated fragment conformer is of low energy, the number of the major points used to obtain the conformer is saved in a table. The major point is saved, even if the low-energy conformer was calculated at a minor point about the major point. If the calculated fragment conformer is of high energy, the conformer is considered unstable and not likely to be a part of a preferred conformer for the molecule, and is discarded. The conformer is considered to be low-energy if it is within N kcals of the global minima; that is, within N kcals of the lowest energy conformation calculated so far for the fragment. N is obtained by multiplying the number of rotatable bonds by a scaling factor, which may range anywhere from 0 to 2.0 kcals/bond. In a current preferable embodiment, N is generally 0.5 kcals/bond. The scaling factor can be higher (e.g., 1.5 kcals/bond) when it is desirable to cover the conformational space more completely.

Once the fragment conformer set has been calculated, it is stored in the database. This makes the conformer set available for future computations that require determination of a conformer set for a fragment derived from a similar molecule, and may also be used in future executions for a totally different molecule, provided that the totally different molecule contains a fragment matching the canonical form of the stored fragment. For example, to determine a conformer set for fragment 803, the previously stored conformer set for fragment 801 may be used, bypassing the repeated calculation of what would be an identical conformer set.

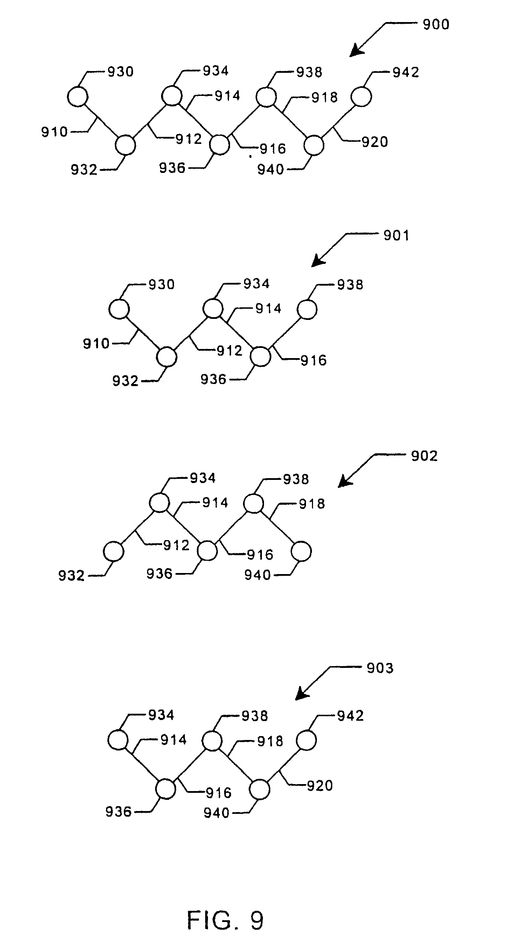

Fig. 9 depicts a graph 900 of a hypothetical molecule being broken into overlapping fragments 901, 902, and 903. Molecule 900 is of atom length 9, with seven nodes shown and omitting representation for additional hydrogen atoms bound to atoms represented by node 930 and node 942 (not shown). Graph 900 comprises seven nodes 930, 932, 934, 936, 938, 940, and 942, and six edges 910, 912, 914, 916, 918, and 920. Fragment 901 comprises five nodes 930, 932, 934, 936, and 938, and four edges 910, 912, 914, and 916. Fragment 902 comprises five nodes 932, 934, 936, 938, and 940, and four edges 912, 914, 916, and 918. Fragment 903 comprises five nodes 934, 936, 938, 940 and 942, and four edges 914, 916, 918, and 920.

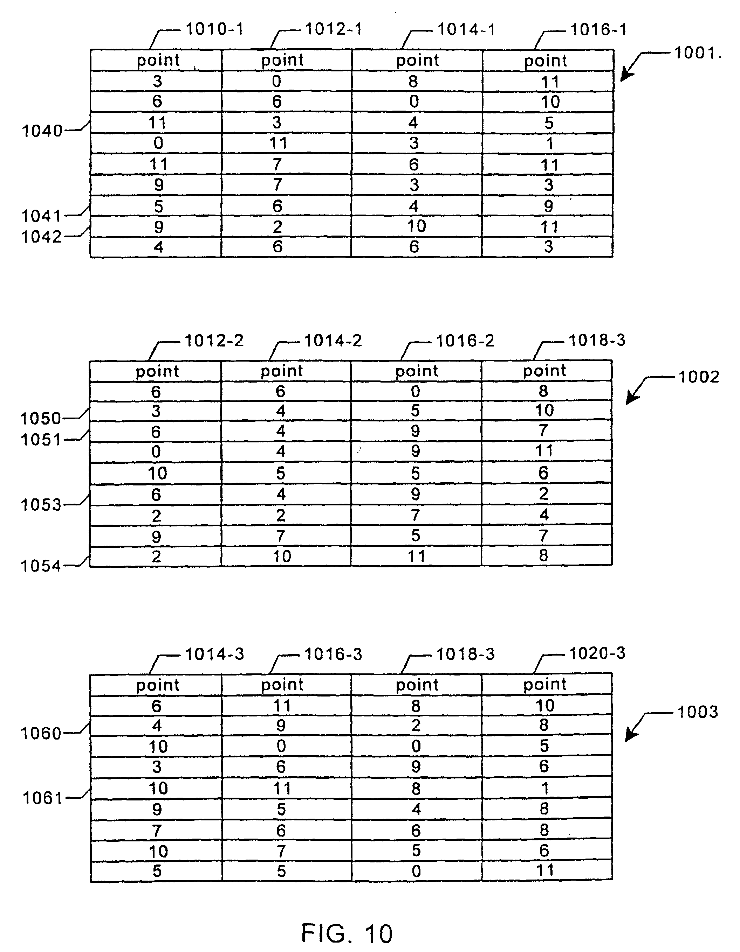

Fig. 10 depicts conformation sets for each of fragments 901, 902, and 903. Tables 1001, 1002, and 1003 each depict a hypothetical conformation set for fragments 901, 902, and 903, respectively. Each table comprises a plurality of columns, each of which corresponds to a edge in the graph fragment, which in turn corresponds to a rotatable bond in the atom being conformationally analyzed. Thus, table 1001, representing a conformation set for fragment 901, comprises columns 1010-1, 1012-1, 1014-1, and 1016-1, corresponding to edges 910, 912, 914, and 916, respectively. Likewise, table 1002, representing a conformation set for fragment 902, comprises columns 1012-2, 1014-2, 1016-2, and 1018-2, corresponding to edges 912, 914, 916, and 918, respectively. Finally, table 1003, representing a conformation set for fragment 903, comprises columns 1014-3, 1016-3, 1018-3, and 1020-32, corresponding to edges 914, 916, 918, and 920, respectively.

Each row in the table represents a conformation for the fragment. For example, table 1001 contains nine rows, indicating nine possible low-energy conformations for fragment 901. The first row in table 1001 contains the sequence 3-0-8-11, which corresponds to a particular possible low energy conformation for fragment 901. This particular sequence indicates a low-energy conformation where the bond corresponding to edge 910 has a torsion measured at point 3, the bond corresponding to edge 912 has a torsion measured at point 0, the bond corresponding to edge 914 has a torsion measured at point 8, and the bond corresponding to edge 916 has a torsion measured at point 11. The particular degree value for the torsion will depend on the nature of the bond (i.e., sp²-sp², sp²-sp³ or sp³-sp³).

The value of the tabular form will become apparent in the discussion of fragment intersection below.

### 4.3 Fragment Intersection

Conformation sets corresponding to two overlapping fragments may be intersected to produce a conformation set corresponding to a larger fragment comprising both fragments. This process may be iterated or combined until the conformation sets for all fragments in the molecule have been intersected. The resulting conformation set represents a conformation set for the entire molecule.

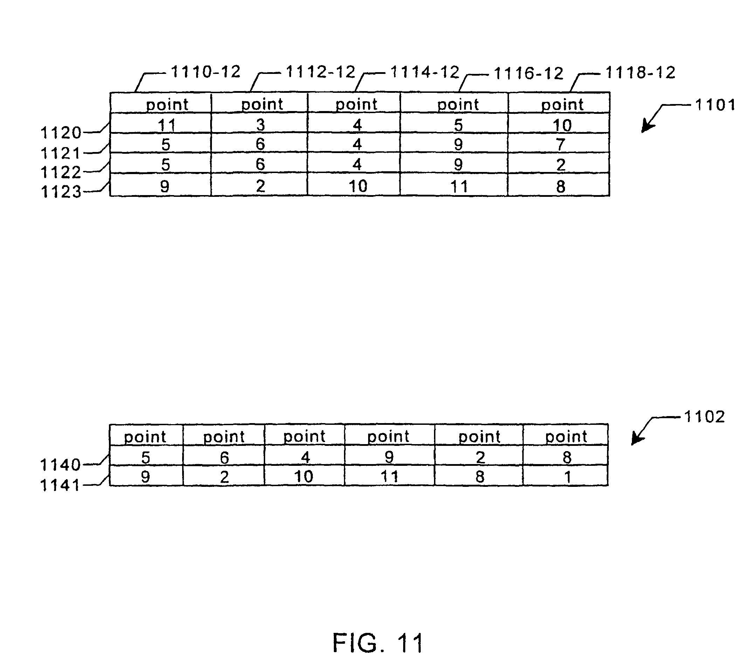

Fig. 11 depicts the intersection of conformation sets of Fig. 10. Table 1101 represents the intersection of table 1001 and table 1002. Column 1012-1 in table 1001 represents a torsion value for the bond corresponding to edge 912 in a conformer for fragment 901. Similarly, column 1012-2 in 1002 represents a torsion value for the bond corresponding to edge 912 in a conformer for fragment 902. A similar relationship is found between column 1014-1 in table 1001 and 1014-2 in table 1002, and between column 1016-1 in table 1001 and 1016-2 in table 1002. That is, each column pair represents a column of torsion values for a particular edge common to both fragments. Columns 1014-1 and 1014-2 correspond to edge 914, and columns 1016-1 and 1016-2 correspond to edge 916.

If a conformer row in table 1001 is found having values for columns 1012-1, 1014-1, and 1016-1 equal to the values for columns 1012-2, 1014-2, and 1016-2 for a conformer row in table 1002, then the corresponding conformers for the two fragments are identical for the bonds shared by the two fragments. Therefore, a new row may be constructed, comprising the shared values and one value from each selected row. The result will be a row whose order is one greater than the order or the rows from which it is derived. For example, if four-column table 1001 has three columns in common with four-column table 1002, the intersection of the table will have five columns: the three common columns and one additional column from each table.

The operation is clearly shown with an example. By inspection of table 1001, it will be seen that row 1040 has values of 3, 4, and 5 for columns 1012-1, 1014-1 and 1016-1, respectively. Likewise, by inspection of table 1002, it will be seen that row 1050 has values 3, 4, and 5 for columns 1012-2, 1014-2, and 1016-2, respectively. Accordingly, an intersection of the two rows may be constructed, having a values of 11, 3, 4, 5, and 10. The value 11 counts from unshared column 1010-1 in row 1040. The values 3, 4, 5 come from common columns 1012-1, and 1012-2, 1014-1, and 1014-2, and 1016-1 and 1016-2 for rows 1040 and 1050. The value 10 comes from unshared column 1018-3 in row 1050.

The resulting sequence 11, 3, 4, 5, 10 represents a low energy conformer for a fragment of length 2-8 comprising bonds 910, 912, 914, 916, and 918.

A complete intersection of tables 1001 and 1002 is depicted as table 1101. Row 1120 represents the operation just described. It will be noted that there are three other rows resulting from the intersection of tables 1001 and 1002. Row 1041 has values for 1012-1, 1014-1 and 1016-1 in common with columns 1012-2, 1014-2, and 1016-2 for rows 1051 and 1053, thus resulting in rows 1121 and 1122 in table 1101. Similarly, rows 1042 and 1054 intersect to form row 1123.

Table 1101, which now describes low energy conformers for an 8 length fragment comprising bonds 910 through 918 may be further intersected with table 1003, which describes low-energy conformers for 7 length fragment 903 comprising bonds 914 through 920. The two tables have three bonds in common. Columns 1014-3, 1016-3, and 1018-3 in table 1003 and columns 1114-12, 1116-12, and 1118-12 in table 1101 each correspond to edges 914, 916, and 918, respectively. Therefore, the two tables may be intersected to form a third table 1102. Table 1102 describes a fragment of length 9 comprising bonds 910, 912, 914, 916, 918, and 920. Row 1122 may be intersected with row 1060 (common values 4, 9, and 2) to form row 1140; row 1123 may be intersected with row 1061 (common values 10, 11, and 8) to form row 1141.

As noted above, table 1102 describes a fragment of length 9 comprising bonds 910, 912, 914, 916, 918, and 920. Referring to Fig. 9, it will be seen that this fragment actually is equivalent to the entire 9 molecule. Thus, with the intersection to form table 1102, conformational analysis of the entire table is complete. That is, table 1102 comprises a list of the preferred conformations for the molecule being analyzed.

It will be noted that the intersection of the tables may be efficiently performed as a relational join of tables in a relational database. In addition, it will be noted that the values in each column are restricted to a range of 0 through 11. This permits each value to be encoded in a 4-bit hexadecimal digit (a half-byte, or "nibble"). The advantage of such encoding is that up to eight values in common may be loaded into a single 32-bit (4-byte) word, as is common to many commonly available inexpensive computers. Consequently, this approach allows for very rapid comparison of up to eight column-rows in a single computer instruction. Although the comparison may be by a COMPARE instruction or the like, in many architectures, a bit-wise comparison may be performed for greater speed. For example, a 32-bit word containing up to eight column-rows from one table may be combined with a 32-bit word containing the corresponding column-rows from another table by means of an exclusive OR operation. A zero result indicates that all column-row values correspond, and a non-zero result indicates that at least one column-row does not correspond. Since exact identification of a non-corresponding set of column-rows is not necessary to the operation of the invention, this approach allows for extremely rapid evaluation of multiple columns to determine whether a particular selection of rows is found to intersect.

### 4.4 Ring Structures

In an alternative embodiment, a method by which ring structures contained within molecular structures can be analyzed will now be described.

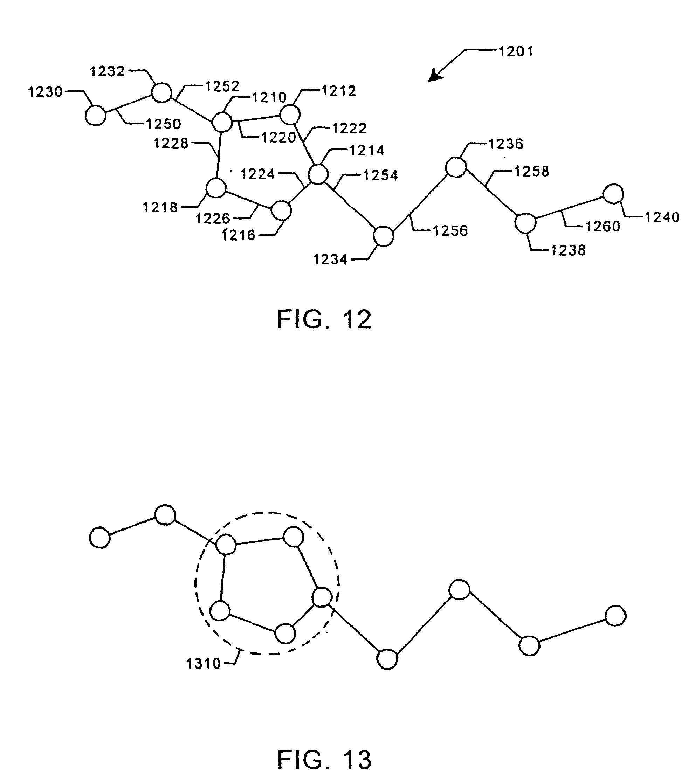

Figure 12 depicts a sample molecule 1201 containing a ring structure. The ring structure comprises nodes (representing atoms) 1210, 1212, 1214, 1216 and 1218 and edges (representing bonds) 1220, 1222, 1224, 1226 and 1228. In addition, nodes 1230, 1232, 1234, 1238, and 1240 and edges 1250, 1252, 1254, 1256, 1258, and 1260 are said to be outside the ring structure.

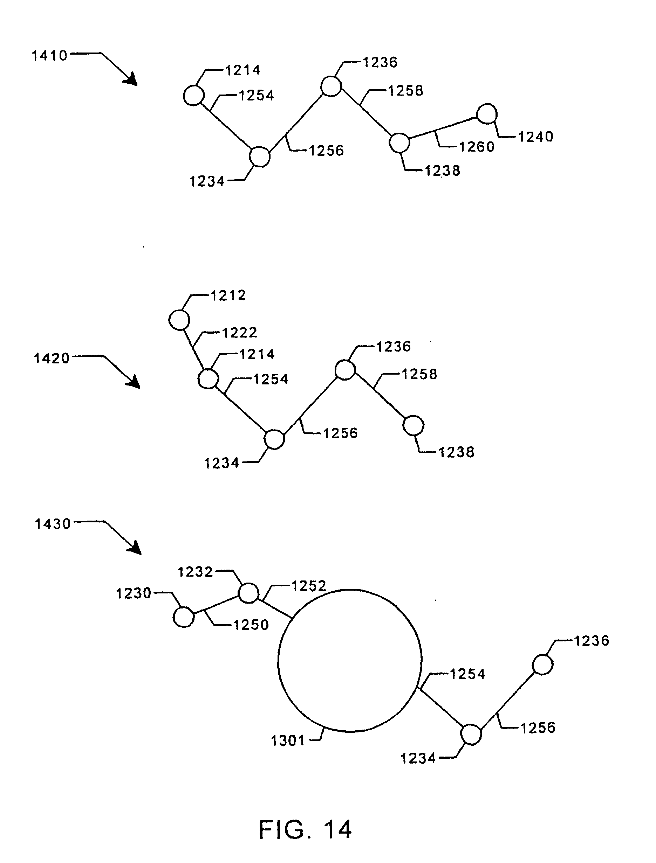

For purposes of decomposition, the ring may be considered a node capable of multiple conformations. In this way, the conformational variation of ring systems can be explored. Figure 13 depicts the demarcation of a ring as a ring node 1310 within molecule 1201. Figure 14 depicts some of the fragments that are formed during decomposition of molecule 1201. Fragments 1410 and 1420 are ordinary linear fragments not containing a ring structure. It will be noted that fragment 1410 comprises one node 1214 within the ring structure, and that fragment 1420 comprises two nodes 1212 and 1214 and one edge 1222 within the ring structure, and that neither of these conditions requires special treatment.

Where paths walk into a ring, special handling is required if any rotatable bond in the path is also a bond in the ring. In this case, a separate algorithm is used to prune the conformations of the path by the conformations of the ring structure. Only conformations of the path that have torsions compatible with torsions in the ring are retained. As described previously, paths are only permitted to walk a maximum of two bonds into a ring, so at most, two torsions from the ring can be used to prune the conformations of the paths.

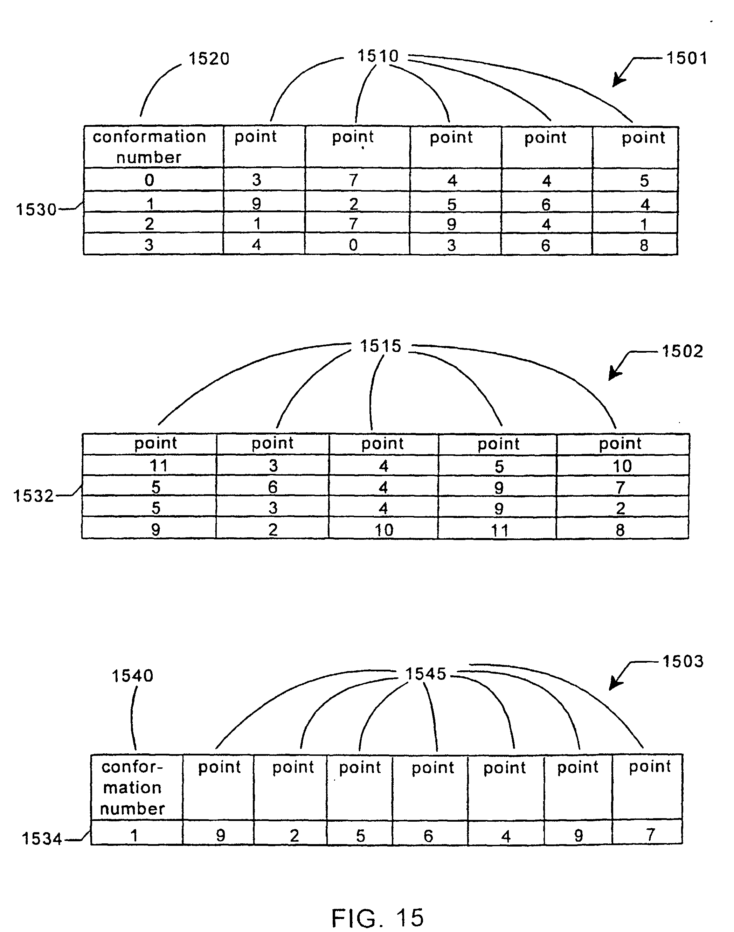

Figure 15 depicts tables used in conformational analysis and intersection of ring nodes. Table 1501 represents a ring node and the conformations that it may assume. Table 1501 contains one row for each potential low-energy conformation that the ring structure, taken alone, could assume. Columns 1510 represent the points corresponding to angles of torsion, just as the columns in fragment conformation sets such as 1001, 1002, and 1003 do. In addition, table 1501 comprises an additional column 1520 that identifies a conformation number associated with the low-energy conformation of the ring.

Table 1502 is a fragment conformation table similar to fragment conformation tables 1001, 1002 and 1003. Table 1502 may be intersected with table 1501 to produce table 1503. Row 1530 in table 1501 contains point values 5, 6, and 4. Likewise, row 1532 in table 1502 contains the same point values 5, 6, and 4. In this example, it is assumed that the point values 5, 6, and 4 in both rows each describe the same edges corresponding to the same molecular bonds. Therefore, the two rows may be intersected to form row 1534 in table 1503.

In such an intersection, the resulting table includes not only the point values from both rows that are pertinent to the new fragment, but also the conformation number of the ring conformation in which the matching values were found. Since the ring is capable of assuming multiple conformations, the inclusion of the conformation number prevents row 1503 from being improperly intersected with a row from another table describing another fragment, where that other table may have the same point values from the ring structure, but from a different conformation.

### 4.5 Torsional Analysis for Resolving Strain in Conformers

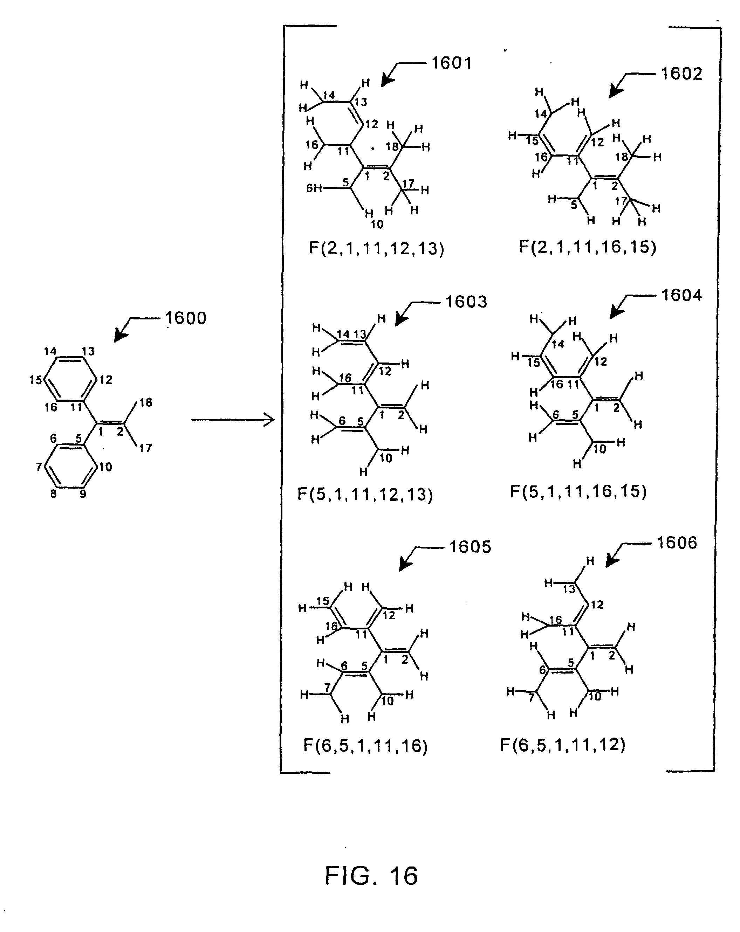

Fig. 16 depicts an example of a molecular structure, 1600, which is computationally decomposed into a plurality of fragments, 1601, 1602, 1603, 1604, 1605 and 1606. The notation F(A1, A2, ...An) is used to represent a fragment derived from a linear atom sequence A1 through An. Each normalized fragment derived from the molecular structure is conformationally analyzed in isolation.

Conformational models of each fragment comprise the torsions of low-energy conformations of the fragments. In a particular embodiment, these conformational models may be represented as a table, where each row represents a conformer of the fragment and each column represents an internal coordinate in the fragment. Here, we use torsions as the internal coordinates. In these tables, central torsions (i.e. the torsions defined by the atoms in the defining path of the fragment) are listed. A global minimum energy value is computed for each conformer for each fragment and stored with the conformer in the table. In a current preferred embodiment, conformers are sorted by relative energy above the global minimum for the fragment. An energy cut-off may be employed in the generation of these fragment conformational models in order to control the number of conformers retained. Conformational energies are measured using a standard molecular mechanics force field which estimates the energy of the conformer as a function of its internal coordinates. In a current embodiment, the DREIDING force field is used in its entirety. For further information regarding the DREIDING technique, reference may be had to Mayo, Olafson and Goddard, J.Phys.Chem (1990) 94, pp8897-8909 which is incorporated herein by reference for all purposes.

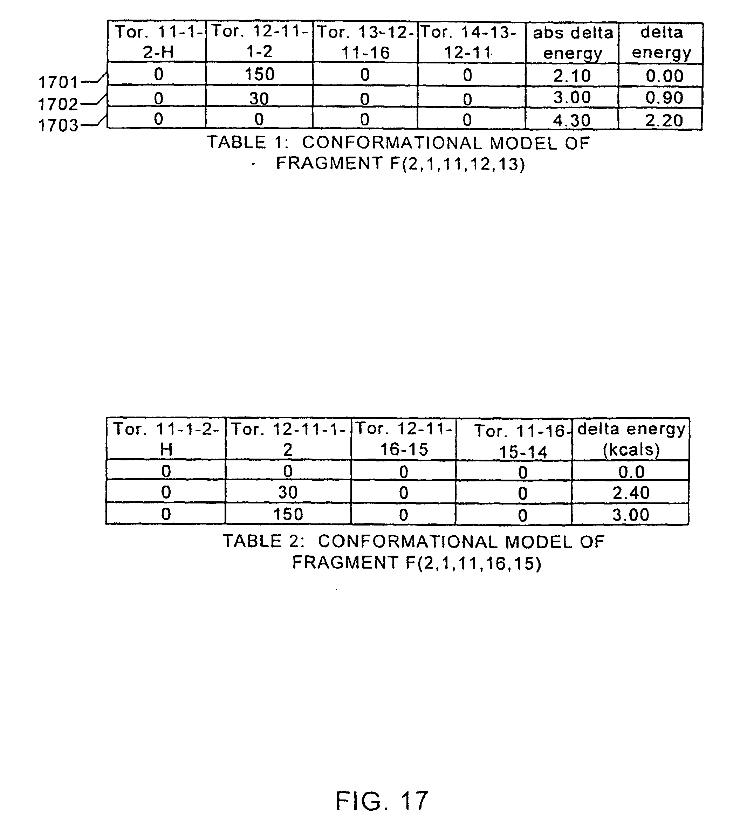

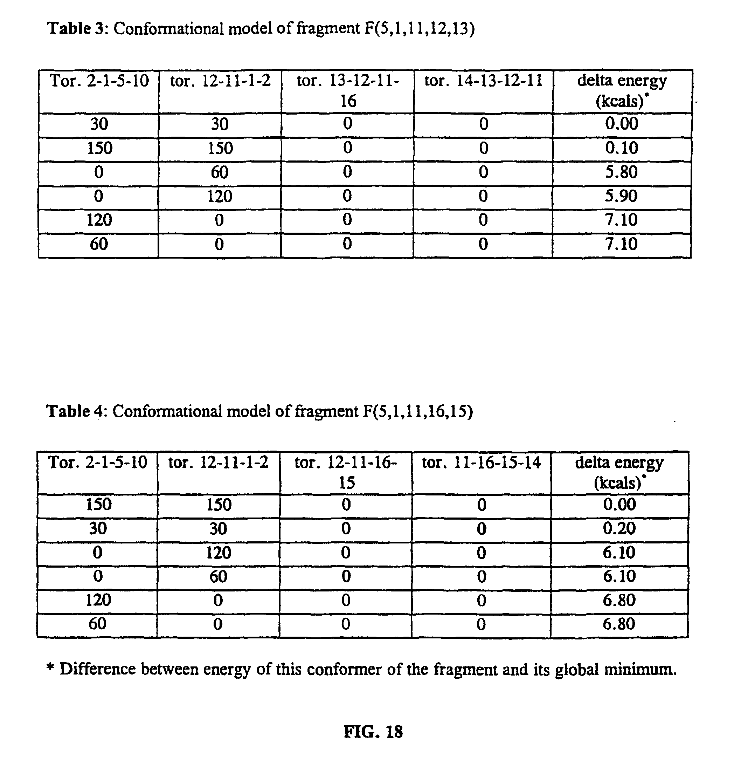

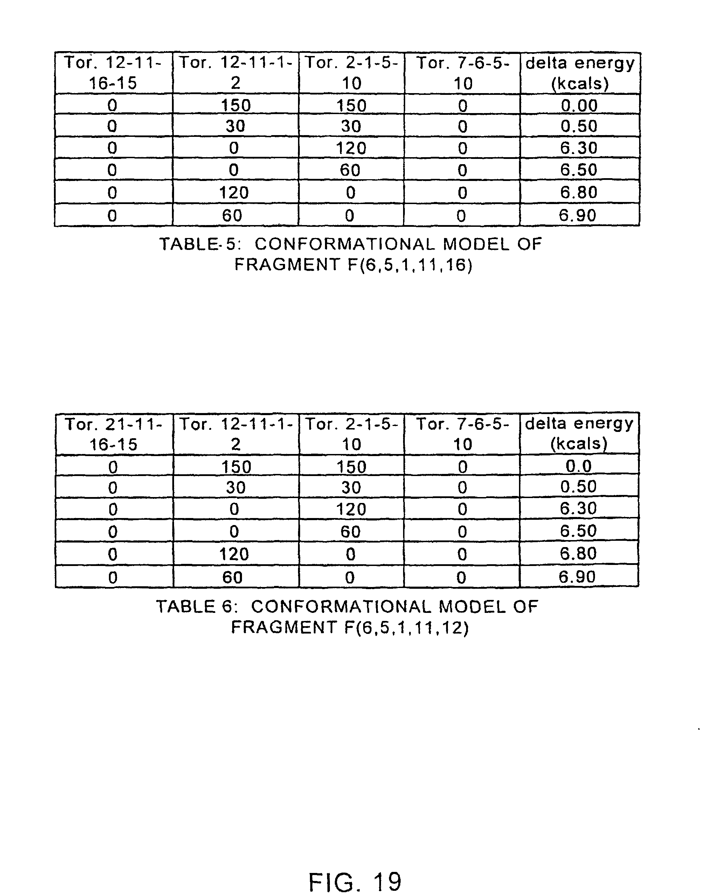

A conformational model of the entire molecular structure is constructed by intersecting the torsion tables of each of the fragments. Tablel - table 6 in Figs. 17 - 19 list the conformational models of the example fragments 1601, 1602, 1603, 1604, 1605 and 1606 shown in Fig. 16.

Certain torsions may be eliminated from consideration because these torsions provide no information about the conformation. For example, torsions that lie within a ring structure have a structure that is completely determined by the conformation of the ring. For example, consider torsions 13-12-11-16 and 14-13-12-11 in Fig. 16. These correspond to fragments that lie exclusively within the phenyl ring of the example, which comprises atoms 11-12-13-14-15-16. Given the complete list of conformations of the fragment F(2,1,11,12,13) in table 1 of Fig. 17, conformers that do not have torsions consistent with the phenyl ring can be eliminated.

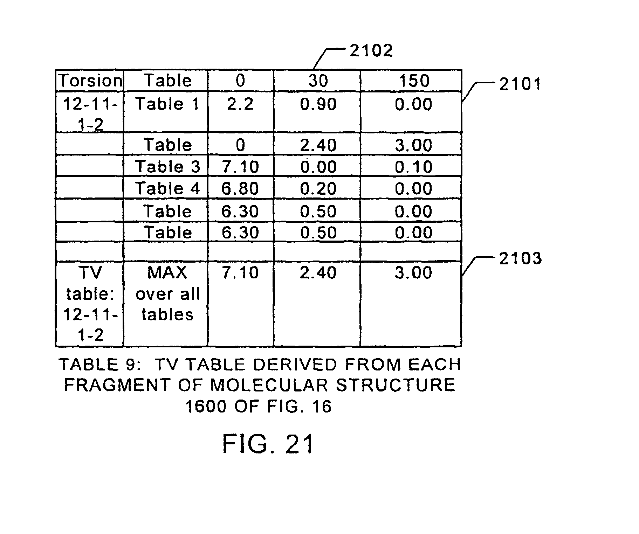

In a particular embodiment, a mechanism of a Fragment-Torsion-Value table ("FTV table") and a subsequent Torsion-Value (T-V table) is employed in order to resolve information about torsion angle strains among the conformers. Fig. 21 depicts a simplified representative FTV table, table 9, having rows, such as a row 2101, representing "variable" torsions and columns, such as a column 2102, indexed by characteristic torsion angle values for each torsion. An FTV table may be constructed incrementally by considering each conformational model of the constituent fragments in sequence. As used herein, a "variable torsion" is any exocyclic torsion crossed by a fragment. In certain embodiments, the FTV table is three dimensional, having a typical entry represented by FTV(f,b,v), wherein f is a particular fragment, b is a particular variable torsion and v is the value of a particular torsion. A separate FTV table can be provided for each fragment, in which each entry is the incremental energy above a particular nominal value, such as a global minimum energy for the fragment, of the first conformer that has the torsion b at the value v. The entry represents the way that this fragment can place a particular torsion at a particular value at the lowest incremental energy.

The quantity TV(v,b) is the maximum of all FTV table entries, FTV(f,v,b), for all fragments f. It is the least strained way of achieving the torsion b at the value v over all fragments f. The simplified FTV table, table 9 of Fig. 21, has entries for an example torsion 12-11-1-2. For every fragment in which this torsion appears, the incremental energy above the global minimum of the first conformer that has this torsion at a particular value v is set in the FTV table. In row 2101 of table 9, the first conformer of the fragment 1601 in table 1, fragment F(2,1,11,12,13), that sets the torsion value to 30 degrees for torsion 12-11-1-2 is 0.9 kcals above the global minimum for the fragment. This may be represented as, when f = F(2,1,11,12,13), b =torsion 12-11-1-2, and v = 30 degrees, FTV(f,b,v) = 0.9. When all such entries are determined for every bond and every value for every fragment, the TV entries for every torsion value may be determined by taking the maximum of each column in table 9.

The TV table entries for any bond, after considering all fragments that cross the bond, express the lowest energy which will be expended in order for any fragment to set the torsion to a particular characteristic angle value. For example, row 2103 of table 9 predicts that torsion 12-11-1-2 prefers values of 30 degrees and 150 degrees and has a particularly large incremental energy when the torsion angle is 0 degrees. At least one fragment, F(6,5,1,11,16), in the molecule possessed the requisite information about a high-energy H-H interaction between the phenyl rings when this torsion is planar. It is this fragment that dominates the entries in the TV table when torsion 12-11-1-2 is planar.

### 4.6 Minimizing Energy of a Conformer's Bonds

The quantity TV(v,b), is the maximum incremental energy required for the first conformer to have torsion b at value v, over all fragments that contain torsion b. For all values v for a particular torsion b, there is one TV entry for every such value v. The TV(v,b) quantities may be derived from the FrV(f,v,b) entries. An example of TV entries for torsion 12-11-1-2 are contained in row 2103 of table 9.

Since each TV(v,b) entry represents the lowest energy state in which some fragment had a torsion b at value v, it is not worth considering values v that require too large an incremental energy above the global minimum for the row. Row 2103 of table 9 indicates that for torsion 12-11-1-2 to have a value of 0 degrees, some fragment's incremental energy must rise to at least 7.1 kcals above it's own global minimum. However, to achieve a value of 30 degrees or 150 degrees, the largest incremental energy that any fragment must have is 2.4 kcals and 3.0 kcals respectively.

Sorting the FTV table by incremental energy can facilitate the selecting of TV values for torsions likely to appear in the final conformational model. All torsion values within a nominal energy threshold can be retained, while those exceeding the threshold are discarded. A current preferable threshold value is 0.4 kcals per rotatable bond in the fragments. In select embodiments, the threshold may be controlled by a user. In the example of table 9, torsions having characteristic angle values of 30 degrees and 150 degrees will be retained. However, the characteristic angle value of 0 degrees is correctly eliminated, because at least one fragment possessed sufficient contextual information to determine that a value of 0 degrees was a poor choice for torsion 12-11-1-2.

The TV table processing limits the characteristic angle values that any torsion can assume. In our example, any conformer of a fragment that does not have a value surviving the cutoff analysis is no longer considered. This is illustrated in table 10, where table 1 and table 5 are intersected at an energy cutoff (for each individual table) of 2.0 kcals. From conformer 1701 of table 1, it can be seen that lowest energy conformation has torsion 12-11-1-2 set to a value of 0 degrees. However, this value was eliminated during the TV table processing, so conformer 1701 is eliminated from further consideration. The new global minimum energy conformer 1702 of table 1 is the second conformer in the table. Conformer 1702 and conformer 1703 are included in subsequent join processing. The result of the intersection of these conformers is depicted in table 11.

### 5.0 Conclusion

In conclusion the present invention provides for a method for dynamically determining conformers for a synthesizable molecular structure based upon information about its constituents. In some embodiments, the present invention provides more automated methods of analyzing molecular structures using a computer than many of the manual techniques heretofore known. The present invention can also provide a library of fragments which is without theoretical limit as to size or scope. Some embodiments according to the invention can consider entire molecular contexts in determining a molecular structure based upon fragment conformers. Select embodiments according to the invention may be more robust than those. heretofore known in the art.

## Claims

1. A computer based method for determining a conformation for a molecular structure comprising the steps of:
automatically decomposing (450) the molecule into a plurality of partially overlapping and normalized (452) fragments, each fragment having a limited maximum number (4) of rotatable bonds, each bond having a number of possible torsion angles;
determining (454) for each of said fragments one or more possible conformers thereof, torsion angles of each possible conformer having values that maximize (462) a plurality of determined (460) minimum energy difference levels;
determining (464) for each of said fragments one or more candidate conformers, said candidate conformers having a range of likely torsion angles broader than the range of torsion angles of said possible conformers and obtained (463) by correspondingly increasing by a predetermined amount said maximized minimum energy difference levels;
for each fragment of the molecule selecting and combining (466) one or more candidate conformers to produce a low energy conformation for the molecular structure;
said method being **characterized in that**
for each one of said partially overlapping fragments (901-903) of the molecule (900) a corresponding table (1001-1003) is provided having a plurality of columns and rows and storing values defining said likely torsion angles of said one or more candidate conformers, each column representing a rotatable bond, each row representing one of said candidate conformers having a possible low-energy conformation of the fragment;
said step of selecting and combining is performed by:
examining the tables (1001, 1002) corresponding to a pair of partially overlapping fragments (901., 902) and selecting rows (1040-1042; 1050-1054) that contain the same values for those rotatable bonds shared by the pair; creating an intersection table (1101) the rows of which combine the values of said selected rows;
likewise examining said intersection table (1101) and the table (1003) corresponding to another partially overlapping fragment, thereby creating a further intersection table (1102);
repeating the above step until all tables for all fragments in the molecule have been examined;
the rows of the finally obtained intersection table representing said low energy conformation for the molecular structure.

2. The method of claim 1 wherein the determining step (454) further comprises:
searching in a library of normalized fragments for at least one matching fragment, said matching fragment being identical to at least one normalized fragment (452) in said plurality of normalized fragments; thereupon,
if said matching fragment is found, using conformer information associated with said matching fragment as said at least one of said plurality of candidate conformers (464), otherwise, computing said at least one of said plurality of conformers of the normalized fragment and storing said at least one of said plurality of fragments in said library.

3. The method of claim 1 or claim 2 wherein fragments are represented by a plurality of nodes and edges.

4. The method of claim 3 wherein each of said plurality of nodes represents a collection of atoms in a molecule.

5. The method of claim 3 wherein each of said plurality of edges represents a molecular bond.

6. The method of any of claims 3 to 5 wherein in said decomposing step the fragments are enumerated based upon a path including nodes and edges, said path having a characteristic length, said characteristic length being the number of atoms in said path, said characteristic length having a minimum value of M, said characteristic length having a maximum value P.

7. The method of claim 6 wherein M is 2 and P is 7.

8. The method of any of claims 1 to 7 wherein determination of said determined (460) minimum energy difference levels includes the step of determining a global minimum energy value for each said fragment.

9. The method of claim 8 wherein said automatically determining said global minimum energy value for said fragment further comprises:
determining a quantity of rotatable bonds in said fragment; and multiplying said quantity by a per bond energy factor.

10. The method of claim 9 wherein said per bond energy factor ranges from 1.0 kcals to 4.0 kcals per rotatable bond.

11. The method of any of claims 1 to 10 wherein said method comprises the step of automatically determining an energy threshold value for said fragment.

12. The method of claim 11 wherein said automatically determining said energy threshold value for said fragment further comprises:
determining a quantity of rotatable bonds in said fragment; and multiplying said quantity by a per bond cut-off energy.

13. The method of claim 12 wherein said per bond cut-off energy is 0.4 kcals per rotatable bond.

14. A computer programming product for determining a conformation for a molecular structure comprising:
code for automatically decomposing (450) the molecule into a plurality of partially overlapping and normalized (452) fragments, each fragment having a limited maximum number (4) of rotatable bonds, each bond having a number of possible torsion angles;
code for determining (454) for each of said fragments one or more possible conformers thereof, torsion angles of each possible conformer having values that maximize (462) a plurality of determined (460) minimum energy difference levels;
code for determining (464) for each of said fragments one or more candidate conformers, said candidate conformers having a range of likely torsion angles broader than the range of torsion angles of said possible conformers and obtained (463) by correspondingly increasing by a predetermined amount said maximized minimum energy difference levels;
code for selecting and combining (466) for each fragment of the molecule one or more candidate conformers to produce a low energy conformation for the molecular structure;
said programming product being **characterized by**
code for providing for each one of said partially overlapping fragments (901-903) of the molecule (900) a corresponding table (1001-1003) having a plurality of columns and rows and storing values defining said likely torsion angles of said one or more candidate conformers, each column representing a rotatable bond, each row representing one of said candidate conformers having a possible low-energy conformation of the fragment;
said code for selecting and combining including :
code for examining the tables (1001, 1002) corresponding to a pair of partially overlapping fragments (901, 902) and selecting rows (1040-1042; 1050-1054) that contain the same values for those rotatable bonds shared by the pair;
code for creating an intersection table (1101) the rows of which combine the values of said selected rows;
code for likewise examining said intersection table (1101) and the table (1003) corresponding to another partially overlapping fragment, thereby creating a further intersection table (1102);
code for repeating the above step until all tables for all fragments in the molecule have been examined;
the rows of the finally obtained intersection table representing said low energy conformation for the molecular structure, and;
a computer readable storage medium for storing the codes.

15. The computer programming product of claim 14 wherein the code for determining (454) further comprises:
code for searching in a library of normalized fragments for at least one matching fragment, said matching fragment being identical to at least one normalized fragment (452) in said plurality of normalized fragments; thereupon,
if said matching fragment is found, using conformer information associated with said matching fragment as said at least one of said plurality of candidate conformers (464), otherwise, computing said at least one of said plurality of conformers of the normalized fragment and storing said at least one of said plurality of fragments in said library.

16. The computer programming product of claim 14 or claim 15 wherein fragments are represented by a plurality of nodes and edges.

17. The computer programming product of claim 16 wherein each of said plurality of nodes represents a collection of atoms in a molecule.

18. The computer programming product of claim 16 wherein each of said plurality of edges represents a molecular bond.

19. The computer programming product of any of claims 16 to 18 wherein in said code for automatically decomposing (454) the molecule further comprises code for enumerating the fragments based upon a path including nodes and edges, said path having a characteristic length said characteristic length being the number of atoms in said path, said characteristic length having a minimum value of M, said characteristic length having a maximum value P.

20. The computer programming product of claim 19 wherein M is 2 and P is 7.

21. The computer programming product of any of claims 14 to 20 wherein said code for determination of said determined (460) minimum energy difference levels includes code for determining a global minimum energy value for each said fragment.

22. The computer programming product of claim 21 wherein said automatically determining said global minimum energy value for said fragment further comprises:
code for determining a quantity of rotatable bonds in said fragment; and multiplying said quantity by a per bond energy factor.

23. The computer programming product of claim 22 wherein said per bond energy factor ranges from 1.0 kcals to 4.0 kcals per rotatable bond.

24. The computer programming product of any of claims 14 to 23 wherein said method comprises the step of automatically determining an energy threshold value for said fragment.

25. The computer programming product of claim 24 wherein said code for automatically determining said energy threshold value for said fragment further comprises:
code for determining a quantity of rotatable bonds in said fragment; and multiplying said quantity by a per bond cut-off energy.

26. The computer programming product of claim 25 wherein said per bond cut-off energy is 0.4 kcals per rotatable bond.

27. An apparatus for determining a conformation for a molecular structure comprising:
means for automatically decomposing (450) the molecule into a plurality of partially overlapping and normalized (452) fragments, each fragment having a limited maximum number (4) of rotatable bonds, each bond having a number of possible torsion angles;
means for determining (454) for each of said fragments one or more possible conformers thereof, torsion angles of each possible conformer having values that maximize (462) a plurality of determined (460) minimum energy difference levels;
means for determining (464) for each of said fragments one or more candidate conformers, said candidate conformers having a range of likely torsion angles broader than the range of torsion angles of said possible conformers and obtained (463) by correspondingly increasing by a predetermined amount said maximized minimum energy difference levels;
means for selecting and combining (466) for each fragment of the molecule one or more candidate conformers to produce a low energy conformation for the molecular structure;
said apparatus being **characterized by**:
means for providing for each one of said partially overlapping fragments (901-903) of the molecule (900) a corresponding table (1001-1003) having a plurality of columns and rows and storing values defining said likely torsion angles of said one or more candidate conformers, each column representing a rotatable bond, each row representing one of said candidate conformers having a possible low-energy conformation of the fragment;
said means for selecting and combining including :
means for examining the tables (1001, 1002) corresponding to a pair of partially overlapping fragments (901, 902) and selecting rows (1040-1042; 1050-1054) that contain the same values for those rotatable bonds shared by the pair, thereby creating an intersection table (1101) the rows of which combine the values of said selected rows;
means for likewise examining said intersection table (1101) and the table (1003) corresponding to another partially overlapping fragment, thereby creating a further intersection table (1102);
means for repeating the above step until all tables for all fragments in the molecule have been examined;
the rows of the finally obtained intersection table representing said low energy conformation for the molecular structure.

28. The apparatus of claim 27 wherein the means for determining (454) conformers further comprises:
means for searching in a library of normalized fragments for at least one matching fragment, said matching fragment being identical to at least one normalized fragment (452) in said plurality of normalized fragments; thereupon,
if said matching fragment is found, using conformer information associated with said matching fragment as said at least one of said plurality of candidate conformers (464), otherwise, computing said at least one of said plurality of conformers of the normalized fragment and storing said at least one of said plurality of fragments in said library.

29. The apparatus of claim 28 or claim 29 wherein fragments are represented by a plurality of nodes and edges.

30. The apparatus of claim 29 wherein each of said plurality of nodes represents a collection of atoms in a molecule.

31. The apparatus of claim 29 wherein each of said plurality of edges represents a molecular bond.

32. The apparatus of any of claims 27 to 31 wherein the means for decomposing comprises means for enumerating fragments based upon a path including nodes and edges, said path having a characteristic length, said characteristic length being the number of atoms in said path, said characteristic length having a minimum value of M, said characteristic length having a maximum value P.

33. The apparatus of claim 32 wherein M is 2 and P is 7.

34. The apparatus of any of claims 27 to 33 wherein the means for determination of said determined (460) minimum energy difference levels includes means for determining a global minimum energy value for each said fragment.

35. The apparatus of claim 34 wherein said means for automatically determining said global minimum energy value for said fragment further comprises:
means for determining a quantity of rotatable bonds in said fragment; and multiplying said quantity by a per bond energy factor.

36. The apparatus of claim 35 wherein said per bond energy factor ranges from 1.0 kcals to 4.0 kcals per rotatable bond.

37. The apparatus of any of claims 27 to 36 wherein said apparatus comprises means for automatically determining an energy threshold value for said fragment.

38. The apparatus of claim 37 wherein said means for automatically determining said energy threshold value for said fragment further comprises:
means for determining a quantity of rotatable bonds in said fragment; and multiplying said quantity by a per bond cut-off energy.

39. The apparatus of claim 38 wherein said per bond cut-off energy is 0.4 kcals per rotatable bond.

## Patentansprüche

1. Rechnergestütztes Verfahren zum Ermitteln einer Konformation für eine Molekülstruktur, wobei zu dem Verfahren die Schritte gehören:
automatisches Zerlegen (450) des Moleküls in mehrere, teilweise überlappende und normierte (452) Fragmente, wobei jedes Fragment eine beschränkte, maximale Anzahl (4) von rotierbaren Bindungen aufweist und jede Bindung eine Anzahl von möglichen Torsionswinkeln besitzt;
für jedes der Fragmente wird eines oder mehrerer seiner möglichen Konformere ermittelt (454), wobei die Torsionswinkel eines jeden möglichen Konformers Werte aufweisen, die mehrere ermittelte (460) minimale Energiedifferenzpegel maximieren (462);
Ermitteln (464) eines oder mehrerer Kandidatenkonformere für jedes der Fragmente, wobei die Kandidatenkonformere einen Bereich von wahrscheinlichen Torsionswinkeln aufweisen, der breiter ist als der Bereich von Torsionswinkeln der möglichen Konformere und der ermittelt wird (463), indem der maximierte minimale Energiedifferenzpegel um einen vorgegebenen Betrag entsprechend erhöht wird;
Auswählen und Kombinieren (466) eines oder mehrerer Kandidatenkonformere für jedes Fragment des Moleküls, um eine energiearme Konformation für die molekulare Struktur hervorzubringen;
wobei das Verfahren **dadurch gekennzeichnet ist, dass**
für jedes einzelne der teilweise überlappenden Fragmente (901-903) des Moleküls (900) eine entsprechende Tabelle (1001-1003) mit mehreren Spalten und Zeilen für die Speicherung von Werten vorgesehen ist, die die wahrscheinliehen Torsionswinkel des einen Konformers oder der Vielzahl von Kandidatenkonformeren definieren, wobei jede Spalte eine rotierbare Bindung repräsentiert und jede Zeile einen der Kandidatenkonformere repräsentiert, die eine mögliche energiearme Konformation des Fragments aufweisen;
der Schritt des Auswählens und Verknüpfens beinhaltet:
dass die Tabellen (1001, 1002) untersucht werden, die einem Paar von teilweise überlappenden Fragmenten (901, 902) entsprechen, und Zeilen (1040-1042; 1050-1054) ausgewählt werden, die die gleichen Werte für diejenigen rotierbaren Bindungen enthalten, die dem Paar gemeinsam sind;
eine Schnittmengentabelle (1101) angelegt wird, deren Zeilen die Werte der ausgewählten Zeilen verknüpfen;
dass die Schnittmengentabelle (1101) und die einem weiteren teilweise überlappenden Fragment entsprechende Tabelle (1003) in ähnlicher Weise untersucht werden und dadurch eine weitere Schnittmengentabelle (1102) erzeugt wird;
dass die obigen Schritte wiederholt werden, bis sämtliche Tabellen für sämtliche in dem Molekül vorkommende Fragmente untersucht sind;
wobei die Zeilen der am Schluss sich ergebenden Schnittmengentabelle die energiearme Konformation für die molekulare Struktur repräsentieren.

2. Verfahren nach Anspruch 1, bei dem der ermittelnde Schritt (454) ferner beinhaltet,
dass eine Bibliothek von normierten Fragmenten nach wenigstens einem passenden Fragment durchsucht wird, wobei dieses passende Fragment mit wenigstens einem normierten Fragment (452) aus der Vielzahl von normierten Fragmenten identisch ist; und dass daraufhin,
falls dieses passende Fragment gefunden wird, die Konformerinformation, die dem passenden Fragment zugeordnet sind, wie dieser wenigstens eine Konformer aus der Vielzahl von Kandidatenkonformeren (464) verwendet wird, anderenfalls das wenigstens eine Konformer aus der Vielzahl von Konformern des normierten Fragments berechnet wird und dieses wenigstens eine Fragment aus der Vielzahl von Fragmenten in der Bibliothek gespeichert wird.

3. Verfahren nach Anspruch 1 oder Anspruch 2, bei dem Fragmente durch eine Reihe von Knoten und Kanten dargestellt sind.

4. Verfahren nach Anspruch 3, bei dem jeder Knoten aus der Vielzahl von Knoten eine Gruppe von Atomen in einem Molekül repräsentiert.

5. Verfahren nach Anspruch 3, bei dem jeder Bogen aus der Vielzahl von Kanten eine Molekülbindung repräsentiert.

6. Verfahren nach einem beliebigen der Ansprüche 3 bis 5, bei dem in dem Zerlegungsschritt die Fragmente auf der Grundlage eines Pfades gezählt werden, der Knoten und Kanten aufweist, wobei der Pfad eine charakteristische Länge aufweist und diese charakteristische Länge gleich der Anzahl der Atome in dem Pfad ist, wobei die charakteristische Länge einen Minimalwert M und die charakteristische Länge einen Maximalwert P aufweist.

7. Verfahren nach Anspruch 6, bei dem M gleich 2 ist und P gleich 7 ist.

8. Verfahren nach einem beliebigen der Ansprüche 1 bis 7, bei dem die Bestimmung des ermittelten (460) minimalen Energiedifferenzpegels den Schritt des Ermittelns einer globalen minimalen Energie für jedes der Fragmente beinhaltet.

9. Verfahren nach Anspruch 8, bei dem zu dem automatischen Ermitteln des globalen minimalen Energiewertes für das Fragment ferner gehören: Ermitteln einer Anzahl von rotierbaren Bindungen in dem Fragment; und Multiplikation dieser Anzahl mit einem Faktor der Energie pro Bindung.

10. Verfahren nach Anspruch 9, bei dem der Faktor der Energie pro Bindung im Bereich von 1,0 kcal bis 4,0 kcal pro rotierbare Bindung liegt.

11. Verfahren nach einem beliebigen der Ansprüche 1 bis 10, bei dem zu dem Verfahren der Schritt eines automatischen Ermittelns eines Energieschwellenwerts für das Fragment gehört.

12. Verfahren nach Anspruch 11, bei dem das automatische Ermitteln des Energieschwellwerts für das Fragment ferner beinhaltet:
Ermitteln der Anzahl von rotierbaren Bindungen in dem Fragment; und Multiplikation dieser Anzahl mit einem Trennenergiefaktor pro Bindung.

13. Verfahren nach Anspruch 12, bei dem der Trennenergiefaktor pro Bindung 0,4 kcal pro rotierbare Bindung beträgt.

14. Computerprogrammierendes Produkt, um eine Konformation für den Aufbau eines Moleküls zu ermitteln, zu dem gehören:
ein Programmkode, der dazu dient, das Moleküls in mehrere teilweise überlappende und normierte (452) Fragmente automatisch zu zerlegen (450), wobei jedes Fragment eine beschränkte maximale Anzahl (4) von rotierbaren Bindungen aufweist, von denen jede eine Anzahl von möglichen Torsionswinkeln aufweist;
ein Programmkode, der dazu dient, für jedes der Fragmente einen oder mehrere seiner möglichen Konformere zu ermitteln (454), wobei die Torsionswinkel eines jeden möglichen Konformers Werte aufweisen, die mehrere ermittelte (460) minimale Energiedifferenzpegel maximieren (462);
ein Programmkode, der dazu dient, für jede der Fragmente ein oder mehrere Kandidatenkonformere zu ermitteln (464), wobei die Kandidatenkonformere einen Bereich von wahrscheinlichen Torsionswinkeln aufweisen, der breiter ist als der Bereich von Torsionswinkeln der möglichen Konformere und durch entsprechendes Erhöhen des maximierten minimalen Energiedifferenzpegels um einen vorgegebenen Betrag ermittelt (463) wird;
ein Programmkode, der dazu dient, für jedes Fragment des Moleküls ein oder mehrere Kandidatenkonformere auszuwählen und zu verknüpfen (466), um eine energiearme Konformation für die molekulare Struktur zu erzeugen;
wobei das Computerprogrammierendes Produkt **dadurch gekennzeichnet ist,**
**dass** es einen Programmkode aufweist, der dazu dient, für jedes einzelne der teilweise überlappenden Fragmente (901-903) des Moleküls (900) eine entsprechende Tabelle (1001-1003) mit mehreren Spalten und Zeilen zu erzeugen und darin Werte zu speichern, die die wahrscheinlichen Torsionswinkel des einen oder der Vielzahl von Kandidatenkonformeren definiert, wobei jede Spalte eine rotierbare Bindung repräsentiert und jede Zeile einen der Kandidatenkonformere repräsentiert, die eine mögliche energiearme Konformation des Fragments aufweisen;
und zu dem Programmkode zum Auswählen und Verknüpfen gehören:
ein Programmkode, der dazu dient, die Tabellen (1001, 1002) zu untersuchen, die einem Paar teilweise überlappender Fragmente (901, 902) entsprechen und Zeilen (1040-1042; 1050-1054) auszuwählen, die die gleichen Werte für diejenigen rotierbaren Bindungen enthalten, die dem Paar gemeinsam sind;
ein Programmkode, der dazu dient, eine Schnittmengentabelle (1101) zu erzeugen, deren Zeilen die Werte der ausgewählten Zeilen verknüpfen;
ein Programmkode, der dazu dient, in ähnlicher Weise die Schnittmengentabelle (1101) und die Tabelle (1003) zu untersuchen, die einem weiteren teilweise überlappenden Fragment entspricht, und hiermit eine weitere Schnittmengentabelle (1102) zu erzeugen;
ein Programmkode, der dazu dient, den obigen Schritt zu wiederholen, bis sämtliche Tabellen für sämtliche Fragmente in dem Molekül untersucht sind;
wobei die Zeilen der schließlich erhaltenen Schnittmengentabelle die energiearme Konformation für die molekulare Struktur repräsentieren, und;
ein von einem Rechner auslesbares Speichermedium, das dazu dient, die Programmkodes zu speichern.

15. Computerprogrammierendes Produkt nach Anspruch 14, bei dem der Programmkode zum Ermitteln (454) ferner beinhaltet:
einen Programmkode, der dazu dient, eine Bibliothek von normierten Fragmenten nach wenigstens einem passenden Fragment zu durchsuchen, wobei das passende Fragment zu wenigstens einem normierten Fragment (452) aus der Vielzahl von normierten Fragmenten identisch ist; wobei der Programmkode anschließend,
falls das passende Fragment gefunden wird, die dem passenden Fragment zugeordnete Konformerinformation wie wenigstens einen aus der Vielzahl von Kandidatenkonformeren (464) verwendet, und anderenfalls das wenigstens eine Konformer aus der Vielzahl von Konformeren des normierten Fragments berechnet und das wenigstens eine Fragment aus der Vielzahl von Fragmenten in der Bibliothek speichert.

16. Computerprogrammierendes Produkt nach Anspruch 14 oder Anspruch 15, bei dem Fragmente durch eine Reihe von Knoten und Kanten repräsentiert werden.

17. Computerprogrammierendes Produkt nach Anspruch 16, bei dem jeder der Vielzahl von Knoten eine Gruppe von Atomen in einem Molekül repräsentiert.

18. Computerprogrammierendes Produkt nach Anspruch 16, bei dem jeder der Vielzahl von Kanten eine Molekülbindung repräsentiert.

19. Computerprogrammierendes Produkt nach einem der Ansprüche 16 bis 18, bei dem in dem Programmkode für das automatische Zerlegen (454) des Moleküls ferner Programmkode für eine Zählung der Fragmente auf der Grundlage eines Pfades enthalten ist, der Knoten und Kanten aufweist, wobei der Pfad eine charakteristische Länge besitzt, die gleich der Anzahl von Atomen auf dem Pfad ist, und die charakteristische Länge einen Minimalwert M und die charakteristische Länge einen Maximalwert P aufweist.

20. Computerprogrammierendes Produkt nach Anspruch 19, bei dem M gleich 2 ist und P gleich 7 ist.

21. Computerprogrammierendes Produkt nach einem der Ansprüche 14 bis 20, bei dem der Programmkode, der dazu dient, den ermittelten (460) minimalen Energiedifferenzpegel zu bestimmen, Programmkode enthält, der dazu dient, eine globale minimale Energie für jedes der Fragmente zu ermitteln.

22. Computerprogrammierendes Produkt nach Anspruch 21, bei dem zu dem automatischen Ermitteln der globalen minimalen Energie für das Fragment ferner gehören: ein Programmkode, der dazu dient, eine Anzahl von rotierbaren Bindungen in dem Fragment zu ermitteln; und dass diese Anzahl mit einem Faktor der Energie pro Bindung multipliziert wird.

23. Computerprogrammierendes Produkt nach Anspruch 22, bei dem der Faktor der Energie pro Bindung im Bereich von 1,0 kcal bis 4,0 kcal pro rotierbare Bindung liegt.

24. Computerprogrammierendes Produkt nach einem der Ansprüche 14 bis 23, bei dem zu dem Verfahren der Schritt eines automatischen Ermittelns eines Energieschwellenwert für das Fragment gehört.

25. Computerprogrammierendes Produkt nach Anspruch 24, bei dem der Programmkode für das automatische Ermitteln des Energieschwellenwerts für das Fragment ferner beinhaltet:
einen Programmkode, der dazu dient, eine Anzahl von rotierbaren Bindungen in dem Fragment zu ermitteln; und dass diese Anzahl mit einer Trennenergie pro Bindung multipliziert wird.

26. Computerprogrammierendes Produkt nach Anspruch 25, bei dem die Trennenergie pro Bindung 0,4 kcal pro rotierbare Bindung beträgt.

27. Eine Einrichtung, die dazu dient, eine Konformation für die Struktur eines Moleküls zu ermitteln, wobei zu der Einrichtung gehören:
Mittel zum automatischen Zerlegen (450) des Moleküls in mehrere teilweise überlappende und normierte (452) Fragmente, wobei jedes Fragment eine beschränkte maximale Anzahl (4) von rotierbaren Bindungen, und jede Bindung eine Anzahl von möglichen Torsionswinkeln aufweist;
Mittel, um für jedes der Fragmente einen oder mehrere seiner möglichen Konformere zu ermitteln (454), wobei die Torsionswinkel jedes möglichen Konformers Werte aufweisen, die mehrere ermittelte (460) minimale Energiedifferenzpegel maximieren (462);
Mittel, um für jedes der Fragmente ein oder mehrere Kandidatenkonformere zu ermitteln (464), wobei die Kandidatenkonformere einen Bereich von wahrscheinlichen Torsionswinkeln aufweisen, der breiter ist als der Bereich von Torsionswinkeln der möglichen Konformere und der durch entsprechendes Erhöhen der maximierten minimalen Energiedifferenzpegel um einen vorgegebenen Betrag berechnet (463) wird;
Mittel, um für jedes Fragment des Moleküls einen oder mehrere Kandidatenkonformere auszuwählen und zu verknüpfen (466), um eine energiearme Konformation für die Molekülstruktur zu erzeugen;
wobei die Einrichtung **gekennzeichnet ist durch**:
Mittel, um für jedes einzelne der teilweise überlappenden Fragmente (901-903) des Moleküls (900) eine entsprechende Tabelle (1001-1003) mit mehreren Spalten und Zeilen zu erzeugen und darin Werte zu speichern, die die wahrscheinlichen Torsionswinkel des einen oder der Vielzahl von Kandidatenkonformeren definiert, wobei jede Spalte eine rotierbare Bindung repräsentiert und jede Zeile einen der Kandidatenkonformere repräsentiert, die eine mögliche energiearme Konformation des Fragments aufweisen;
wobei zu den Mitteln zum Auswählen und Verknüpfen gehören:
Mittel, um die Tabellen (1001, 1002) zu untersuchen, die einem Paar teilweise überlappender Fragmente (901, 902) entsprechen, und Zeilen (1040-1042; 1050-1054) auszuwählen, die die gleichen Werte für diejenigen rotierbaren Bindungen enthalten, die dem Paar gemeinsam sind, und hierdurch eine Schnittmengentabelle (1101) zu erzeugen, deren Zeilen die Werte der ausgewählten Zeilen verknüpfen;
Mittel, um in ähnlicher Weise die Schnittmengentabelle (1101) und die Tabelle (1003) zu untersuchen, die einem weiteren teilweise überlappenden Fragment entspricht, und hiermit noch eine Schnittmengentabelle (1102) zu erzeugen;
Mittel, um den obigen Schritt zu wiederholen, bis sämtliche Tabellen für sämtliche Fragmente in dem Molekül untersucht sind;
wobei die Zeilen der schließlich erhaltenen Schnittmengentabelle die energiearme Konformation für die molekulare Struktur repräsentieren.

28. Einrichtung nach Anspruch 27, bei dem zu dem Mittel zum Ermitteln (454) von Konformeren ferner gehören:
Mittel, um eine Bibliothek von normierten Fragmenten nach wenigstens einem passenden Fragment zu durchsuchen,
wobei das passende Fragment zu wenigstens einem normierten Fragment (452) aus der Vielzahl von normierten Fragmenten identisch ist; wobei das Mittel anschließend,
falls das passende Fragment gefunden wurde, die dem passenden Fragment zugeordnete Konformerinformation wie wenigstens einen aus der Vielzahl von Kandidatenkonformeren (464) verwendet, und anderenfalls den wenigstens einen aus der Vielzahl von Konformeren des normierten Fragments berechnet und das wenigstens eine aus der Vielzahl von Fragmenten in der Bibliothek speichert.

29. Einrichtung nach Anspruch 28 oder Anspruch 29, bei der Fragmente durch eine Reihe von Knoten und Kanten repräsentiert werden.

30. Einrichtung nach Anspruch 29, bei der jeder aus der Vielzahl von Knoten eine Gruppe von Atomen in einem Molekül repräsentiert.

31. Einrichtung nach Anspruch 29, bei der jeder Bogen aus der Vielzahl von Kanten eine Molekülbindung repräsentiert.

32. Einrichtung nach einem der Ansprüche 27 bis 31, bei der das Mittel zum Zerlegen, Mittel enthält, um Fragmente auf der Grundlage eines Knoten und Kanten aufweisenden Pfades zu zählen, wobei der Pfad eine charakteristische Länge aufweist und diese charakteristische Länge gleich der Anzahl der Atome in dem Pfad ist, wobei die charakteristische Länge einen Minimalwert M und die charakteristische Länge einen Maximalwert P besitzt.

33. Einrichtung nach Anspruch 32, bei der M gleich 2 ist und P gleich 7 ist.

34. Einrichtung nach einem der Ansprüche 27 bis 33, bei der das Mittel zur Bestimmung des ermittelten (460) minimalen Energiedifferenzpegels Mittel zum Ermitteln einer globalen minimalen Energie für jedes der Fragmente einschließt.

35. Einrichtung nach Anspruch 34, bei der zu dem Mittel zum automatischen Ermitteln des globalen minimalen Energiewertes für das Fragment ferner gehören:
Mittel, um eine Anzahl von rotierbaren Bindungen in dem Fragment zu ermitteln; und dass diese Anzahl mit einem Faktor der Energie pro Bindung multipliziert wird.

36. Einrichtung nach Anspruch 35, bei der der Faktor der Energie pro Bindung im Bereich von 1,0 kcal bis 4,0 kcal pro rotierbare Bindung liegt.

37. Einrichtung nach einem der Ansprüche 27 bis 36, bei der die Einrichtung Mittel zum automatischen Ermitteln eines Energieschwellenwerts für das Fragment enthält.

38. Einrichtung nach Anspruch 37, bei der das Mittel zum automatischen Ermitteln des Energieschwellenwerts für das Fragment ferner beinhaltet:
Mittel, um eine Anzahl von rotierbaren Bindungen in dem Fragment zu ermitteln; und dass diese Anzahl mit einem Trennenergiefaktor pro Bindung multipliziert wird.

39. Einrichtung nach Anspruch 38, bei der der Trennenergiefaktor pro Bindung 0,4 kcal pro rotierbare Bindung beträgt.

## Revendications

1. Procédé de détermination de la conformation d'une structure moléculaire basé sur l'informatique comprenant les étapes consistant à :
décomposer automatiquement (450) la molécule en une pluralité de fragments se chevauchant partiellement et normalisés (452), chaque fragment ayant un nombre limité maximum (4) de liaisons rotatives, chaque liaison ayant un nombre d'angles de torsion possibles ;
déterminer (454) pour chacun desdits fragments un ou plusieurs conformères possibles de ceux-ci, les angles de torsion de chaque conformère possible ayant des valeurs qui optimisent (462) une pluralité de niveaux de différence d'énergie minimum déterminés (460) ;
déterminer (464) pour chacun desdits fragments un ou plusieurs conformères candidats, lesdits conformères candidats ayant une plage d'angles de torsion probables plus large que la plage des angles de torsion desdits conformères possibles et obtenus (463) en augmentant en conséquence d'une quantité prédéterminée lesdits niveaux de différence d'énergie minimum optimisés ;
pour chaque fragment de la molécule, sélectionner et combiner (466) un ou plusieurs conformères candidats pour produire une conformation à faible énergie pour la structure moléculaire ;
ledit procédé étant **caractérisé en ce que** pour chacun desdits fragments se chevauchant partiellement (901-903) de la molécule (900), un tableau correspondant (1001-1003) est prévu, ayant une pluralité de colonnes et de lignes et enregistrant des valeurs définissant lesdits angles de torsion probables desdits un ou plusieurs conformères candidats, chaque colonne représentant une liaison rotative, chaque ligne représentant un desdits conformères candidats ayant une conformation de fragment possible à faible énergie ;
ladite étape de sélection et de combinaison est réalisée en :
examinant les tableaux (1001;1002) correspondant à une paire de fragments se chevauchant partiellement (901, 902) et sélectionnant les lignes (1040-1042 ; 1050-1054) qui contiennent les mêmes valeurs pour ces liaisons rotatives partagées par la paire ; créant un tableau d'intersection (1101) dont les lignes combinent les valeurs desdites lignes sélectionnées ;
examinant de la même manière ledit tableau d'intersection (1101) et le tableau (1003) correspondant à un autre fragment se chevauchant partiellement, créant ainsi un autre tableau d'intersection (1102) ;
répétant l'étape précédente jusqu'à ce que tous les tableaux pour tous les fragments dans la molécule aient été examinés ;
les lignes du tableau d'intersection obtenues finalement représentant ladite conformation à faible énergie pour la structure moléculaire.

2. Procédé selon la revendication 1, dans lequel l'étape de détermination (454) comprend en outre :
la recherche dans une librairie de fragments normalisés d'au moins un fragment concordant, ledit fragment concordant étant identique à au moins un fragment normalisé (452) dans ladite pluralité de fragments normalisés ; puis,
si on trouve ledit fragment concordant, l'utilisation de données du conformère associées au dit fragment concordant comme l'un au moins dit de ladite pluralité de conformères candidats (464), sinon la compilatisation dudit au moins un de ladite pluralité de conformères du fragment normalisé et l'enregistrement dudit au moins un de ladite pluralité de fragments dans ladite librairie.

3. Procédé selon la revendication 1 ou 2, où les fragments sont représentés par une pluralité de noeuds et d'arêtes.

4. Procédé selon la revendication 3, où chacune de ladite pluralité de noeuds représente une collection d'atomes dans une molécule.

5. Procédé selon la revendication 3, dans lequel chacune de ladite pluralité d'arêtes représente une liaison moléculaire.

6. Procédé selon l'une quelconque des revendications 3 à 5, où dans ladite étape de décomposition, les fragments sont énumérés sur la base d'un chemin incluant des noeuds et des arêtes, ledit chemin ayant une longueur caractéristique, ladite longueur caractéristique étant le nombre d'atomes dans ledit chemin, ladite longueur caractéristique ayant une valeur minimum de M, ladite longueur caractéristique ayant une valeur maximum P.

7. Procédé selon la revendication 6, où M est 2 et P est 7.

8. Procédé selon l'une quelconque des revendications 1 à 7, où la détermination desdits niveaux de différence d'énergie minimum déterminés (460) comprend l'étape consistant à déterminer une valeur d'énergie minimum totale pour chaque dit fragment.

9. Procédé selon la revendication 8, où ladite détermination automatique de ladite valeur d'énergie minimum totale pour ledit fragment comprend en outre :
la détermination d'une quantité de liaisons rotatives dans ledit fragment et la multiplication de ladite quantité par un facteur d'énergie par liaison.

10. Procédé selon la revendication 9, où le facteur d'énergie par liaison va de 1,0 kcal à 4,0 kcal par liaison rotative.

11. Procédé selon l'une des revendications 1 à 10, ledit procédé comprenant l'étape consistant à déterminer automatiquement une valeur seuil d'énergie pour ledit fragment.

12. Procédé selon la revendication 11, où ladite détermination automatique de ladite valeur seuil d'énergie pour ledit fragment comprend en outre :
la détermination d'une quantité de liaisons rotatives dans ledit fragment et la multiplication de ladite quantité par une énergie de coupure par liaison.

13. Procédé selon la revendication 12, où ladite énergie de coupure par liaison est de 0,4 kcal par liaison rotative.

14. Produit de programmation informatique de détermination de la conformation d'une structure moléculaire comprenant :
un code de décomposition automatique (450) de la molécule en une pluralité de fragments se chevauchant partiellement et normalisés (452), chaque fragment ayant un nombre maximum limité (4) de liaisons rotatives, chaque liaison ayant un nombre d'angles de torsion possibles ;
un code de détermination (454) pour chacun desdits fragments d'un ou de plusieurs conformères possibles de ceux-ci, les angles de torsion de chaque conformère possible ayant des valeurs qui optimisent (462) une pluralité de niveaux de différence d'énergie minimum déterminés (460) ;
un code de détermination (464) pour chacun desdits fragments d'un ou plusieurs conformères candidats, lesdits conformères candidats ayant une plage d'angles de torsion probables plus large que la plage d'angles de torsion desdits conformères possibles et obtenus (463) en augmentant de manière correspondante d'une quantité prédéterminée lesdits niveaux de différence d'énergie minimum optimisés ;
un code de sélection et de combinaison (466) pour chaque fragment de la molécule d'un ou de plusieurs conformères candidats pour produire une conformation à faible énergie pour la structure moléculaire ;
ledit produit de programmation étant **caractérisé en ce que** le code pour fournir pour chacun desdits fragments se chevauchant partiellement (901-903) de la molécule (900) un tableau correspondant (1001-1003) ayant une pluralité de colonnes et de lignes et pour enregistrer des valeurs définissant lesdits angles de torsion probables desdits un ou plusieurs conformères candidats, chaque colonne représentant une liaison rotative, chaque ligne représentant l'un desdits conformères candidats ayant une conformation à faible énergie possible du fragment ;
ledit code de sélection et de combinaison comprenant :
le code d'examen des tableaux (1001, 1002) correspondant à une paire de fragments se chevauchant partiellement (901, 902) et de sélection des lignes (1040-1042 ; 1050-1054) qui contiennent les mêmes valeurs pour ces liaisons rotatives partagées par la paire ;
le code de création d'un tableau d'intersection (1101) dont les lignes combinent les valeurs desdites lignes sélectionnées ;
le code pour examiner de la même manière ledit tableau d'intersection (1101) et le tableau (1003) correspondant à un autre fragment se chevauchant partiellement, créant ainsi un autre tableau d'intersection (1102) ;
le code de répétition de l'étape précédente jusqu'à ce que tous les tableaux pour tous les fragments dans la molécule aient été examinés ;
les lignes du tableau d'intersection obtenu finalement représentant ladite conformation à faible énergie pour la structure moléculaire, et ;
un moyen d'enregistrement lisible informatique pour enregistrer les codes.

15. Produit de programmation informatique selon la revendication 14, où le code de détermination (454) comprend en outre :
le code de recherche dans une librairie de fragments normalisés pour au moins un fragment concordant, ledit fragment concordant étant identique à au moins un fragment normalisé (452) dans ladite pluralité de fragments normalisés ; puis
si ledit fragment concordant est trouvé, l'utilisation des informations du conformère associées au dit fragment concordant comme au moins un dit de ladite pluralité de conformères candidats (464), sinon, la compilation d'un au moins dit de ladite pluralité de conformères de fragment normalisé et l'enregistrement d'au moins un de ladite pluralité de fragments dans ladite librairie.

16. Produit de programmation informatique selon la revendication 14 ou la revendication 15, où les fragments sont représentés par une pluralité de noeuds et d'arêtes.

17. Produit de programmation informatique selon la revendication 16, où chacune de ladite pluralité des noeuds représente une collection d'atomes dans une molécule.

18. Produit de programmation informatique selon la revendication 16, où chacune de ladite pluralité d'arêtes représente une liaison moléculaire.

19. Produit de programmation informatique selon l'une quelconque des revendications 16 à 18, dans ledit code de décomposition automatique (454) de la molécule comprend en outre le code d'énumération des fragments sur la base d'un chemin incluant des noeuds et des arêtes, ledit chemin ayant une longueur caractéristique, ladite longueur caractéristique étant le nombre d'atomes dans ledit chemin, ladite longueur caractéristique ayant une valeur minimum de M, ladite longueur caractéristique ayant une valeur maximum P.

20. Produit de programmation informatique selon la revendication 19, où M est 2 et P est 7.

21. Produit de programmation informatique selon l'une quelconque des revendications 14 à 20, où ledit code de détermination desdits niveaux de différence d'énergie minimum déterminés (460) comprend le code de détermination d'une valeur d'énergie minimum globale pour chaque dit fragment.

22. Produit de programmation informatique selon la revendication 21, où ladite détermination automatique de ladite valeur d'énergie minimum globale pour ledit fragment comprend en outre : le code de détermination d'une quantité de liaisons rotatives dans ledit fragment et de multiplication de ladite quantité par un facteur d'énergie par liaison.

23. Produit de programmation informatique selon la revendication 22, où ledit facteur d'énergie par liaison va de 1,0 kcal à 4,0 kcal par liaison rotative.

24. Produit de programmation informatique selon l'une quelconque des revendications 14 à 23, où ladite méthode comprend l'étape de détermination automatique d'une valeur d'énergie seuil pour ledit fragment.

25. Produit de programmation informatique selon la revendication 24, où ledit code de détermination automatique de ladite valeur d'énergie seuil pour ledit fragment comprend en outre :
le code de détermination d'une quantité de liaisons rotatives dans ledit fragment et de multiplication de ladite quantité par une énergie de coupure par liaison.

26. Produit de programmation selon la revendication 25, dans lequel ladite énergie de coupure par liaison est de 0,4 kcals par liaison rotative.

27. Appareil de détermination d'une conformation pour une structure moléculaire comprenant :
un moyen de décomposition automatique (450) de la molécule en une pluralité de fragments se chevauchant partiellement et normalisés (452), chaque fragment ayant un nombre maximum limité (4) de liaisons rotatives, chaque liaison ayant un nombre d'angles de torsion possibles ;
un moyen de détermination (454) pour chacun desdits fragments d'un ou de plusieurs conformères de ceux-ci possibles, des angles de torsion de chaque conformèrepossible ayant des valeurs qui optimisent (462) une pluralité de niveaux de différence d'énergie minimum déterminés (460) ;
un moyen de détermination (464) pour chacun desdits fragments d'un ou de plusieurs conformères candidats, lesdits conformères candidats ayant une plage d'angles de torsion probables plus larges que la plage des angles de torsion desdits conformères possibles et obtenus (463) en augmentant de manière correspondante d'une quantité prédéterminée lesdits niveaux de différence d'énergie minimum optimisés ;
un moyen de sélection et de combinaison (466) pour chaque fragment de la molécule d'un ou de plusieurs conformères candidats pour produire une conformation à faible énergie pour la structure moléculaire ;
ledit appareil étant **caractérisé par** :
un moyen de dotation pour chacun desdits fragments se chevauchant partiellement (901-903) de la molécule (900) d'un tableau correspondant (1001-1003) ayant une pluralité de colonnes et de lignes et d'enregistrement des valeurs définissant lesdits angles de torsion probables dudit un ou plusieurs conformères candidats, chaque colonne représentant une liaison rotative, chaque ligne représentant un desdits conformères candidats ayant une conformation du fragment à faible énergie possible ;
un moyen de sélection et de combinaison comprenant :
un moyen d'examen des tableaux (1001,1002) correspondant à une paire de fragments se chevauchant partiellement (901, 902) et de sélection de lignes (1040-1042 ; 1050 - 1054) qui contiennent les mêmes valeurs pour ces liaisons rotatives partagées par la paire, créant ainsi un tableau d'intersection (1101) dont les lignes combinent les valeurs desdites lignes sélectionnées ;
un moyen d'examen similaire dudit tableau d'intersection (1101) et du tableau (1003) correspondant à un autre fragment se chevauchant partiellement, créant ainsi un autre tableau d'intersection (1102) ;
un moyen de reproduction de l'étape précédente jusqu'à ce que tous les tableaux pour tous les fragments dans la molécule aient été examinés ;
les lignes du tableau d'intersection finalement obtenues représentant ladite conformation à faible énergie pour la structure moléculaire.

28. Appareil selon la revendication 27, où le moyen de détermination (454) des conformères comprend en outre :
un moyen de recherche dans une librairie de fragments normalisés d'au moins un fragment concordant, ledit fragment concordant étant identique à au moins un fragment normalisé (452) dans ladite pluralité de fragments normalisés ; puis,
si ledit fragment concordant est trouvé, l'utilisation des informations de conformère associées au dit fragment concordant comme au moins l'un de ladite pluralité de conformères candidats (464) sinon, la compilation dudit au moins un de ladite pluralité de conformères du fragment normalisé et l'enregistrement dudit au moins un de ladite pluralité de fragments dans ladite librairie.

29. Appareil selon la revendication 28 ou 29, où les fragments sont représentés par une pluralité de noeuds et d'arêtes.

30. Appareil selon la revendication 29, où chacune de ladite pluralité de noeuds représente une collection d'atomes dans une molécule.

31. Appareil selon la revendication 29, où chacune de ladite pluralité d'arêtes représente une liaison moléculaire.

32. Appareil selon l'une quelconque des revendications 27 à 31 où le moyen de décomposition comprend un moyen d'énumération des fragments basé sur un chemin comprenant des noeuds et des arêtes, ledit chemin ayant une longueur caractéristique, ladite longueur caractéristique étant le nombre d'atomes dans ledit chemin, ladite longueur caractéristique ayant une valeur minimum de M, ladite longueur caractéristique ayant une valeur maximum P.

33. Appareil selon la revendication 32, où M est 2 et P est 7.

34. Appareil selon l'une quelconque des revendications 27 à 33, où le moyen de détermination desdits niveaux de différence d'énergie minimum déterminés (460) comprend un moyen de détermination d'une valeur d'énergie minimum globale pour chacun dit fragment.

35. Appareil selon la revendication 34, où ledit moyen de détermination automatique de ladite valeur d'énergie minimale pour ledit fragment comprend en outre :
un moyen de détermination d'une quantité de liaisons rotatives dans ledit fragment et de multiplication de ladite quantité par un facteur d'énergie par liaison.

36. Appareil selon la revendication 35, où ledit facteur d'énergie par liaison va de 1,0 kcal à 4,0 kcal par liaison rotative.

37. Appareil selon l'une quelconque des revendications 27 à 36, où ledit appareil comprend un moyen de détermination automatique d'une valeur seuil d'énergie pour ledit fragment.

38. Appareil selon la revendication 37, où ledit moyen de détermination automatique de ladite valeur seuil d'énergie pour ledit fragment comprend en outre :
un moyen de détermination d'une quantité de liaisons rotatives dans ledit fragment et de multiplication de ladite quantité par une énergie de coupure par liaison.

39. Appareil selon la revendication 38, où ladite énergie de coupure par liaison est de 0,4 kcal par liaison rotative.
